# EUROPEAN PATENT SPECIFICATION

(11) **EP 4 153 302 B1**
(45) Date of publication and mention of the grant of the patent: **29.05.2024**
(21) Application number: 21729598.9
(22) Date of filing: 19.05.2021
(51) Int. Cl.: A61P 11/00, A61P 31/12

(54) **TREATMENT FOR VIRAL RESPIRATORY INFECTIONS**
BEHANDLUNG VON VIRALEN ATEMWEGSINFEKTIONEN
TRAITEMENT POUR INFECTIONS RESPIRATOIRES VIRALES

(30) Priority: 19.05.2020 GB 202007404
(43) Date of publication of application: 29.03.2023
(73) Proprietor: Biosirius Ltd, Dorset DT11 7QD (GB)
(72) Inventor: NASSER, Syed Muhammad Tahir, Farnham Surrey GU9 8LA (GB)
(74) Representative: Dehns
(86) International application number: PCT/GB2021/051207
(87) International publication number: WO 2021/234381

(56) References cited:
- WO-A1-2012/085015
- WO-A1-2015/032932
- WO-A1-2016/139297
- VAN DER SLUIJS KOENRAAD F. ET AL: "Enhanced viral clearance in interleukin-18 gene-deficient mice after pulmonary infection with influenza A virus", IMMUNOLOGY, vol. 114, no. 1, 1 January 2005 (2005-01-01), pages 112-120, XP055827453, GB ISSN: 0019-2805, DOI: 10.1111/j.1365-2567.2004.02000.x Retrieved from the Internet: URL:https://www.ncbi.nlm.nih.gov/pmc/artic les/PMC1782065/pdf/imm0114-0112.pdf>
- Mcgonagle Dennis: "The Role of Cytokines including Interleukin-6 in COVID-19 induced Pneumonia and Macrophage Activation Syndrome-Like Disease", , 3 April 2020 (2020-04-03), XP055802837, Retrieved from the Internet: URL:https://www.ncbi.nlm.nih.gov/pmc/artic les/PMC7195002/pdf/main.pdf [retrieved on 2021-05-10]
- YONG XIONG ET AL: "Transcriptomic characteristics of bronchoalveolar lavage fluid and peripheral blood mononuclear cells in COVID-19 patients", EMERGING MICROBES & INFECTIONS, vol. 9, no. 1, 1 January 2020 (2020-01-01) , pages 761-770, XP055758661, DOI: 10.1080/22221751.2020.1747363
- GIRARD-GUYONVARC'H CHARLOTTE ET AL: "Unopposed IL-18 signaling leads to severe TLR9-induced macrophage activation syndrome in mice", BLOOD, vol. 131, no. 13, 29 March 2018 (2018-03-29), pages 1430-1441, XP055827899, US ISSN: 0006-4971, DOI: 10.1182/blood-2017-06-789552 Retrieved from the Internet: URL:https://ashpublications.org/blood/arti cle/131/13/1430/36633/Unopposed-IL-18-sign aling-leads-to-severe-TLR9>
- SATIS HASAN ET AL: "Prognostic value of interleukin-18 and its association with other inflammatory markers and disease severity in COVID-19", CYTOKINE, ACADEMIC PRESS LTD, PHILADELPHIA, PA, US, vol. 137, 29 September 2020 (2020-09-29), XP086388042, ISSN: 1043-4666, DOI: 10.1016/J.CYTO.2020.155302 [retrieved on 2020-09-29]

## Description

The present invention relates to pharmaceutical compositions for use in the treatment of viral respiratory infections. More specifically, the present invention relates to IL-18 antagonists and their use in the treatment of viral respiratory infections.

Numerous viral infections affect the upper or lower respiratory tract. Respiratory infections can be classified by the causative virus (e.g., coronavirus, influenza virus) or according to syndrome caused (e.g., the common cold, bronchiolitis, croup, pneumonia). Although specific viral pathogens typically cause characteristic clinical manifestations (e.g., rhinovirus typically causes the common cold, respiratory syncytial virus (RSV) typically causes bronchiolitis), each can cause many of the viral respiratory syndromes.

The severity of viral respiratory illness can vary widely depending on the viral pathogen and patient characteristics, e.g. age, sex, health status etc. For many infections, severe disease is more likely in older patients and infants. Morbidity may result directly from viral infection or may be indirect, due to exacerbation of underlying conditions, such as cardiopulmonary conditions, or subsequent bacterial superinfection of the lung, paranasal sinuses, or middle ear.

The COVID-19 pandemic has caused health and economic problems on a global scale. Coronavirus Disease 2019 (COVID-19) is caused by severe acute respiratory syndrome coronavirus 2 (SARS-CoV2). Infection with SARS-CoV2 has been described as having two phases, with the majority of individuals experiencing only the first phase. The first phase consists of a flu-like illness, with sufferers describing intermittent fevers, lethargy and a new onset continuous cough. The second phase, starting at around 5-7 days of symptoms is characterised by sudden onset shortness of breath that becomes progressively worse. It is at this stage that patients usually present in the hospital and a significant proportion of those patients require invasive ventilation and intensive care support.

In the absence of an effective treatment that can prevent the transition to the second phase of the disease and/or relieve the acute respiratory symptoms of those patients in the second phase, there has been a relatively high proportion of patients requiring invasive ventilation. This has necessitated the enforcement of strict social distancing measures to limit the rate of infection, thereby ensuring healthcare services are not overwhelmed with patients with the severe form of COVID-19. However, these measures are likely to have long-term social and economic impacts and there is a need for new effective treatments for COVID-19.

In work leading up to the present invention, the inventor has used autopsy histopathological findings, bronchoalveolar lavage fluid cellular analysis and peripheral blood flow cytometric analysis of SARS-CoV2 positive patients to identify the underlying reasons that explain the pathogenesis of severe COVID-19, its clinical presentation, risk factors and demographic mortality pattern. The inventor has unexpectedly determined that interleukin-18 (IL-18) represents a central node in the immunological nexus of COVID-19 immunopathology. Whilst not wishing to be bound by theory, the inventor has identified a link between the immunopathology of patients severely affected by COVID-19 and the immunopathology of patients with metabolic syndrome and associated disorders, such as coronary artery disease, hypertension and diabetes, particularly hypertension. In particular, it is hypothesised that COVID-19 may expose an inherent weakness in the innate immune system due to metabolic syndrome and associated disorders, or as occur in the course of normal ageing. For instance, it is thought that natural killer cell dysfunction, super-antigen presentation and activation of the inflammasome, resulting in IL-18 cleavage to its active form, may result in a positive feedback cycle of inflammation.

Accordingly, blockade of IL-18 activity is expected to prevent the development of the catastrophic immunopathology characteristic of COVID-19, particularly in subjects with an underlying pathology associated with increased levels of free IL-18 and/or a deficiency of natural killer cells and/or a deficiency of functional natural killer cells, such as metabolic syndrome. Moreover, the immunological model of COVID-19 described in the Examples indicates that IL-18 represents a therapeutic target for viral respiratory infections generally in such subjects.

WO 2015/032932 describes the use of IL-18BP or anti-IL-18 antibodies for use in treating inflammatory diseases and disorders associated with IL-18.

Accordingly, the invention provides an IL-18 antagonist for use in treating a viral respiratory infection in a subject having metabolic syndrome, coronary artery disease, hypertension, atherosclerosis, type II diabetes or a combination thereof, wherein the IL-18 antagonist is:
(a) a mammalian or viral IL-18 binding protein that binds free IL-18; or
(b) an antibody that binds specifically to free IL-18.

The term "viral respiratory infection" as used herein refers to an infection of the lower and optionally the upper respiratory tract with a viral pathogen, i.e. an infection with a viral respiratory pathogen. Thus, a viral respiratory infection is an infection of the bronchial tubes and/or lungs with a viral pathogen.

The term "viral respiratory syndrome" as used herein refers to a set of medical signs (e.g. patient temperature, blood pressure etc.) and symptoms (e.g. cough, shortness of breath etc.) associated with a particular viral respiratory infection and/or a disease or disorder caused or triggered by the viral respiratory infection, e.g. viral pneumonia.

As described in more detail in the Examples, the inventor has determined that some viral respiratory infections, such as SARS-CoV2 infection, can trigger a systemic increase in levels of free IL-18 in the infected subject. Whilst not wishing to be bound by theory, it is hypothesised that the increase in free IL-18, particularly due to a failure of IL-18 Binding Protein (IL-18BP) stimulation and/or release, may underlie clinical features of patients that present with a severe manifestation of the viral respiratory infection, e.g. multi-organ failure, lymphopenia etc. As explained in more detail in the Examples, it is thought that subjects with dysfunctional natural killer cells (e.g. as a result of a genetic condition or an acquired disorder, such as metabolic syndrome or increasing age) are unable to release interferon gamma (IFN-gamma), which is the key promoter of IL-18BP expression, in sufficient quantity so as to stimulate the release of sufficient quantities of IL-18BP in the severe form of the disease. Thus, the viral respiratory infection is an infection by a viral pathogen that can trigger a systemic increase in levels of free IL-18 in the infected subject, i.e. an increase in the blood levels of free IL-18.

Numerous viral pathogens are known to infect the respiratory tract and any such viral pathogen may result in a viral respiratory infection as defined above. In some embodiments, the viral respiratory infection is an infection by a coronavirus, influenza virus, human parainfluenza virus, respiratory syncytial virus, rhinovirus, human metapneumovirus, human bocavirus or adenovirus.

In some embodiments, the influenza virus is an influenza A virus or an influenza B virus.

In some preferred embodiments, the viral respiratory infection is an infection by a coronavirus. In some embodiments, the coronavirus is a severe acute respiratory syndrome (SARS) coronavirus (e.g. SARS-CoV or SARS-CoV2) or a Middle East respiratory syndrome (MERS) coronavirus. In a particularly preferred embodiment, the coronavirus is SARS coronavirus 2 (SARS-CoV2).

Thus, alternatively viewed, in some embodiments the viral respiratory infection is Coronavirus Disease 2019 (COVID-19), SARS or MERS.

In some embodiments, the viral respiratory infection causes a viral respiratory syndrome, such as viral pneumonia, viral bronchitis or viral bronchiolitis.

The terms "subject" and "patient" are used interchangeably herein and refer to a mammal, preferably a human. In particular, the terms subject and patient refer to a human having a viral respiratory infection, as defined herein, and metabolic syndrome, coronary artery disease, hypertension, atherosclerosis, type II diabetes or a combination thereof, in need of treatment. The subject may have deficiencies in interferon-gamma release as a result of metabolic syndrome, coronary artery disease, hypertension, atherosclerosis, diabetes (e.g. type 2 diabetes) or a combination thereof. In a particular embodiment, subjects may have a deficiency as described above as a result of hypertension.

The term "deficiency of IL-18 binding protein" refers to a subject having an amount of IL-18BP in the blood that is lower than the amount of IL-18BP in the blood of a healthy control subject or a subject with the same viral respiratory infection that has a less severe manifestation thereof. As noted above, a deficiency of IL-18 binding protein results in an increase in the levels of free IL-18. A subject having a deficiency of IL-18 binding protein may be viewed as a subject having a high level of free IL-18, i.e. having an amount of free IL-18 in the blood that is higher than the amount of free IL-18 in the blood of a healthy control subject or a subject with the same viral respiratory infection that has a less severe manifestation thereof. Thus, the subject may have a deficiency of IL-18 binding protein and/or a high level of free IL-18.

The amount of IL-18BP and/or free IL-18 in blood of a subject with the same viral respiratory infection that has a less severe manifestation thereof may be a reference value. In some embodiments, the reference value is adjusted based on the characteristics of the subject, e.g. age, sex and/or ethnicity. Thus, the amount of IL-18BP and/or free IL-18 in a blood sample obtained from the subject may be compared to a reference value obtained from a subject with the same viral respiratory infection that has a less severe manifestation thereof or the same or similar (i.e. comparable) characteristics, e.g. age, sex and/or ethnicity. For instance, a comparable control subject may be of the same sex, ethnicity and same age bracket as the subject being tested, e.g. 40-49, 50-59, 60-69 years old etc.

A deficiency of IL-18BP refers to a subject having an amount of IL-18BP in a blood sample obtained from the subject that is at least 5% lower than the reference value, e.g. at least 10, 15, 20, 25, 30% or lower than the reference value, such as at least 50, 75 or 100% lower than the reference value.

A high level of free IL-18 refers to a subject having an amount of free IL-18 in a blood sample obtained from the subject that is at least 5% higher than the reference value, e.g. at least 10, 15, 20, 25, 30% or higher than the reference value, such as at least 50, 75 or 100% higher than the reference value.

Methods for assaying the amount of IL-18BP and/or free IL-18 in a sample from a subject are well-known in the art. Any suitable method for assaying the amount of free IL-18 in a sample from a subject may be used. Such methods for assaying the amount of free IL-18 in a sample from a subject may be an immunoassay, such as an enzyme-linked immunosorbent assay (ELISA) and radio-immunoassay (RIA), which are routinely used in laboratories.

The amount of IL-18BP and/or free IL-18 typically is assayed in a blood sample.

Natural killer (NK) cells are lymphocytes of the innate immune system that mediate cytotoxicity and produce cytokines after the ligation of germline-encoded activation receptors. As a result, they have long been considered part of the innate immune system. NK cells function primarily in the innate defense against viral infections and in tumor cell surveillance but also participate in immunoregulation, coordination of immunity, and modulation of autoreactivity.

NK cells are lymphocytes and major members of the innate lymphoid cell family, which develop from CD341 hematopoietic cells in the bone marrow and undergo terminal maturation in secondary lymphoid tissues. In humans NK cells are classically identified by the absence of the T-cell receptor complex and the presence of neural cell adhesion molecule (denoted CD56 according to the cluster designation system). The majority of peripheral blood NK cells express low levels of CD56 as well as an IgG Fc receptor FcgRIIIA (CD16). A minority of peripheral blood NK cells express high levels of CD56 without expressing CD16 and are considered to comprise a developmentally immature but functionally enabled NK cell subset.

The term "deficiency of natural killer (NK) cells" refers to a subject having an amount of NK cells in the blood that is lower than the amount of NK cells in the blood of a healthy control subject or a subject with the same viral respiratory infection that has a less severe manifestation thereof.

After activation, NK cells are capable of three main functions of immune defense. The first and best characterized is the ability to mediate contact-dependent killing of target cells. This involves the mobilization of highly specialized organelles in NK cells known as lytic granules that contain the pore-forming molecule perforin and death-inducing enzymes, such as granzymes. Once a killing program is triggered in an NK cell, the lytic granules are transported to the interface formed with the targeted cell, and their contents are secreted onto it. This function of cytotoxicity can be accessed by NK cell activation receptors as an innate immune defense or by recognition of IgG-opsonized cells through CD16 to enable antibody-dependent cell-mediated cytotoxicity (ADCC). Through ADCC, NK cells have an intimate interface with adaptive immunity and also enable the functions of certain therapeutic mAbs.

The second function of NK cells is the production of soluble factors to promote direct anti-disease effects, as well as to further induce or regulate immunity. These include a wide variety of cytokines, chemokines, and other regulators. In particular, functional NK cells produce IFN-gamma.

The third function of NK cells is that of promoting and regulating immunity through contact-dependent costimulatory and regulatory mechanisms. NK cells express or can be induced to express a large number of relevant costimulatory and regulatory ligands and can localize to key immunoregulatory sites, including secondary lymphoid tissues in which these contact dependent contributions to immune responses can be affected.

The term "deficiency of functional natural killer (NK) cells" refers to a subject having an NK cells in the blood that have defective NK cell activity, e.g. one or more of the activities mentioned above. Alternatively viewed, the activity of NK cells in the blood is lower than the activity of NK cells in the blood of a healthy control subject or a subject with the same viral respiratory infection that has a less severe manifestation thereof.

Methods for assaying the activity of NK cells in a sample from a subject are well-known in the art and any of the activities mentioned above may be used to assess the activity of NK cells in a sample. NK cell activity can be determined by measuring the ability of the NK cells from a sample to mediate contact-dependent killing of target cells and/or the ability of NK cells to secrete IFN-gamma. The method for assaying the activity of NK cells in a sample from a subject may be an immunoassay to measure the amount of IFN-gamma released from the cells. The activity of NK cells typically is assayed using cells obtained from a blood sample.

Thus, the amount of IFN-gamma in a blood sample may be used a proxy for NK cell activity.

Deficiencies in IL-18BP, NK cells and/or functional NK cells may be acquired as a result of ageing and/or other disorders, such as metabolic syndrome, coronary artery disease, hypertension, atherosclerosis, diabetes (e.g. type 2 diabetes) or a combination thereof.

Thus, the invention provides an IL-18 antagonist for use in treating a viral respiratory infection in a subject having metabolic syndrome, coronary artery disease, hypertension, atherosclerosis, type II diabetes or a combination thereof, e.g. in a subject having hypertension, wherein the IL-18 antagonist is:
(a) a mammalian or viral IL-18 binding protein that binds free IL-18; or
(b) an antibody that binds specifically to free IL-18.

The term "metabolic syndrome" typically refers to subjects having more than one condition, e.g. at least three conditions, selected from abdominal obesity, high blood pressure (hypertension), high blood sugar (e.g. diabetes), high serum triglycerides, and low serum high-density lipoprotein (HDL). Thus, in some embodiments, the subject to be treated has more than one condition selected from abdominal obesity, high blood pressure, high blood sugar (e.g. diabetes), high serum triglycerides, and low serum high-density lipoprotein (HDL). As abdominal obesity is a key sign of metabolic syndrome, in some preferred embodiments, the subject to be treated has abdominal obesity and one or more conditions (e.g. 2, 3 or 4 conditions) selected from high blood pressure, high blood sugar (e.g. diabetes), high serum triglycerides, and low serum high-density lipoprotein (HDL). In some embodiments, the subject to be treated has high blood pressure and one or more conditions (e.g. 2, 3 or 4 conditions) selected from abdominal obesity, high blood sugar (e.g. diabetes), high serum triglycerides, and low serum high-density lipoprotein (HDL).

The term "abdominal obesity" refers to a condition where excessive abdominal fat around the stomach and abdomen has built up to the extent that it is likely to have a negative impact on health. This is also known as central obesity and truncal obesity.

The terms "high blood pressure" and "hypertension" are used interchangeably herein and refer to a long-term medical condition in which the blood pressure in the arteries is persistently elevated. For adults, high blood pressure is present if the resting blood pressure is persistently at or above 130/80 or 140/90 mmHg.

High blood pressure may be classified as primary (essential) hypertension or secondary hypertension. Primary hypertension refers to high blood pressure due to nonspecific lifestyle factors (e.g. excess salt in the diet, excess body weight, smoking, and alcohol use) and genetic factors. Secondary hypertension refers to high blood pressure due to an identifiable cause, such as chronic kidney disease, narrowing of the kidney arteries, an endocrine disorder, or the use of birth control pills. In some embodiments, the subject to be treated has primary hypertension.

Subjects with metabolic syndrome have an increased risk of various disorders, particularly hypertension, diabetes mellitus type 2 and coronary artery disease. However, subjects with hypertension, diabetes mellitus type 2 and/or coronary artery disease do not necessarily also have metabolic syndrome.

Thus, in some embodiments, the subject to be treated has hypertension, diabetes mellitus type 2, atherosclerosis and/or coronary artery disease without metabolic syndrome. However, in some embodiments, the subject to be treated has metabolic syndrome and optionally one or more other conditions selected from hypertension, diabetes mellitus type 2, coronary artery disease, atherosclerosis and a combination thereof.

"Atherosclerosis" is a disease in which the inside of an artery narrows due to the build-up of plaque and can result in coronary artery disease, stroke, peripheral artery disease, or kidney problems, depending on which arteries are affected.

"Coronary artery disease" (CAD) refers to the reduction of blood flow to the heart muscle due to build-up of plaque in the arteries of the heart, i.e. atherosclerosis of the heart arteries. Thus, CAD is also known as atherosclerotic heart disease, coronary heart disease (CHD) or ischemic heart disease (IHD).

"Diabetes mellitus type 2", also known as "type 2 diabetes" (T2D) and adult-onset diabetes, is a form of diabetes that is characterized by high blood sugar, insulin resistance, and relative lack of insulin.

The terms "treating" or "treatment" as used herein refer broadly to any effect or step (or intervention) beneficial in the management of a clinical condition or disorder. Treatment therefore may refer to reducing, alleviating, ameliorating, slowing the development of, or eliminating one or more symptoms of the viral respiratory infection or syndrome that is being treated, relative to the symptoms prior to treatment, or in any way improving the clinical status of the subject. A treatment may include any clinical step or intervention which contributes to, or is a part of, a treatment programme or regimen.

A treatment may include delaying, limiting, reducing or preventing the onset of one or more symptoms of the viral respiratory infection or syndrome, for example relative to the symptom prior to the treatment. Thus, treatment explicitly includes both absolute prevention of occurrence or development of symptoms of the viral respiratory infection or syndrome, and any delay in the development of the viral respiratory infection or syndrome or symptom thereof, or reduction or limitation on the development or progression of the viral respiratory infection or syndrome or symptom thereof.

As discussed further in the Examples, it is thought that blockade of free IL-18 functions to rebalance the inflammatory state of the patient as well as the innate immune system, thereby enabling the body to clear the viral infection. Thus, the term "treatment" does not necessarily imply cure or complete abolition or elimination of the viral respiratory infection or syndrome or symptoms thereof.

Thus, in some embodiments, treating the subject may be viewed as treating the immunopathology in the subject with a viral respiratory infection.

As noted above, some subjects infected with a coronavirus, such as SARS CoV2, may exhibit only symptoms associated with the first phase of the infection, e.g. intermittent fevers, lethargy and a new onset continuous cough, and may be characterised as having a "mild" infection, e.g. mild COVID-19. Such patients may recover without the need for treatment. However, for subjects having an underlying condition as defined above, e.g. an acquired deficiency of IL-18BP, NK cells and/or functional NK cells, such as in metabolic syndrome, that increases the risk of developing a severe form of the viral respiratory syndrome caused by the viral respiratory infection, it may advantageous to treat the subjects in the first phase of the infection to prevent the onset of the second phase of the infection, i.e. the severe form of the disease. Thus, in some embodiments, treating the subject may be viewed as preventing a systemic, pathological reaction to the viral respiratory infection.

Thus, in some embodiments, the invention provides an IL-18 antagonist for use in treating a viral respiratory infection in a subject having metabolic syndrome, coronary artery disease, hypertension, atherosclerosis, type II diabetes or a combination thereof to prevent the development of a severe form of a viral respiratory syndrome caused by the viral respiratory infection, wherein the IL-18 antagonist is:
(a) a mammalian or viral IL-18 binding protein that binds free IL-18; or
(b) an antibody that binds specifically to free IL-18.

However, in some embodiments, the subject to be treated has a severe form of the viral respiratory syndrome caused by the viral respiratory infection as defined above. In some particularly preferred embodiments, the subject to be treated has severe manifestation of COVID-19, i.e. symptoms associated with the second phase of the disease, e.g. shortness of breath and/or the need for invasive ventilation.

Thus, in some embodiments, the viral respiratory infection is severe Coronavirus Disease 2019 (COVID-19).

In some embodiments, the subject to be treated may have other characteristics associated with an increased risk of developing a severe form of a viral respiratory syndrome caused by the viral respiratory infection.

For instance, the age, ethnicity and/or sex of the subject may be indicative of an increased risk of developing a severe form of a viral respiratory syndrome (e.g. COVID-19), particularly in combination with an underlying condition as described above.

In a representative embodiment, subjects with a high risk of developing a severe form of a viral respiratory syndrome (e.g. COVID-19) include subjects with one or more of the following characteristics: (i) male; (ii) aged 50 years old or above, e.g. 55, 60, 65 years old or above; and/or (iii) African or Indo-Pakistani Asian ethnicity. These characteristics, particularly age, are associated with increased levels of IL-18, principally due to worsening innate immune system function, healthy functioning of which is required for IL-18 Binding Protein stimulation.

Thus, in some embodiments, the subject may have an amount of free IL-18 in the blood that is higher than the amount of free IL-18 in the blood of a subject with the same viral respiratory infection that has a less severe manifestation thereof.

In some embodiments, the subject to be treated is at least 50 years old, e.g. 55, 60, 65 years old or above (i.e. an ageing subject). In some particular embodiments, the subject selected for a preventative treatment, i.e. to prevent the development of a severe form of a viral respiratory syndrome caused by the viral respiratory infection, is at least 50 years old, e.g. 55, 60, 65 years old or above.

Methods for assaying the amount of free IL-18 in a sample from a subject are well-known in the art (e.g. WO 2016/139297). Any suitable method for assaying the amount of free IL-18 in a sample from a subject, such as those disclosed in WO 2016/139297 may be used. A method for assaying the amount of free IL-18 in a sample from a subject may be an immunoassay, such as an enzyme-linked immunosorbent assay (ELISA) and radio-immunoassay (RIA), which are routinely used in laboratories. The amount of free IL-18 typically is assayed in a blood sample.

The term "blood sample" as used herein may refer to whole blood or a component or derivative thereof, e.g. peripheral whole blood or a component or derivative thereof. The blood sample may be whole blood, serum or plasma. The blood sample is diluted whole blood, which is a derivative of whole blood. The blood sample may be maintained in the presence of an anticoagulant such as heparin, sodium citrate or ethylene diamine tetra acetic acid (EDTA).

Interleukin-18 (IL-18), also known as interferon-gamma inducing factor, is a cytokine produced by activated macrophages, Kupffer cells and other cells. IL-18 binds to the IL-18 receptor (IL-18R) and induces cell-mediated immunity. Defects (e.g. knock-out) of the IL-18 receptor or IL-18 lead to impaired natural killer (NK) cells activity and impaired Th1 responses. The term "IL-18" as used herein typically refers to human IL-18.

The terms "IL-18 antagonist" or "IL-18 inhibitor" are used interchangeably herein and refer to mammalian or viral IL-18 binding proteins that bind free IL-18 or antibodies that bind specifically to free IL-18 that are capable of directly inhibiting, reducing or blocking the activity or function of IL-18, e.g. IL-18 signalling. In some embodiments, the IL-18 antagonist disrupts the interaction between IL-18 and its receptor. Thus, the IL-18 antagonist directly inhibits, reduces or blocks the activity or function of IL-18.

In some embodiments, a combination of IL-18 antagonists may be used to effect inhibition or blockage of IL-18 activity or signalling.

The terms "agent", "compound", and "active" may be used interchangeably herein to refer to a substance that induces a desired pharmacological and/or physiological effect. The terms also encompass pharmaceutically acceptable and pharmacologically active forms thereof, including salts. The desired effect is the inhibition or blockage of IL-18 activity or signaling.

The IL-18 antagonist is a mammalian or viral IL-18 binding protein that binds free IL-18 or an antibody that binds specifically to free IL-18.

Proteins that bind to and inhibit IL-18 are well-known in the art. For instance, IL-18 Binding Protein (IL-18BP) is a high affinity endogenous receptor that effectively neutralizes circulating IL-18 in a one-to-one ratio. Moreover, numerous antibodies that bind to IL-18 have been developed and any such antibody may find utility in the treatments described herein. In particular, IL-18 antibodies are described in WO 2012/085015 and WO 2016/139297 and may find utility in the treatments described herein. Moreover, methods for producing antibodies that specifically bind to a target protein are well-known in the art and described further below.

While native mammalian IL-18 binding proteins (e.g. human isoforms a-d, SEQ ID NOs: 1-4) exist as an endogenous means for controlling the effects of IL-18 *in vivo,* numerous infectious agents, e.g. viruses, have evolved IL-18 binding proteins. For instance, IL-18 binding proteins have been identified in Molluscum contagiosum virus subtype 1 (MC51 L, MC53L, and MC54L, e.g. GenBank accession number CAB89814.1, SEQ ID NO: 5), Vaccinia virus (e.g. GenBank accession number CAB89842.1, SEQ ID NO: 6), Ectromelia virus (e.g. GenBank accession number CAB89805.1, SEQ ID NO: 7), Lumpy skin disease virus (e.g. GenBank accession number AAK43555.1, SEQ ID NO: 8) and Cowpox virus (e.g. GenBank accession number ARB50252.1, SEQ ID NO: 9).

Thus, in some embodiments, the IL-18 antagonist is a mammalian IL-18 binding protein (IL-18BP), preferably a human IL-18 binding protein, or an IL-18-binding fragment or variant thereof (e.g. an isoform of IL-18BP). In some embodiments, the IL-18 antagonist is recombinant human IL-18BP. In some embodiments, the IL-18 antagonist is a viral IL-18 binding protein or an IL-18-binding fragment or variant thereof.

Thus, in some embodiments, the IL-18 binding protein comprises:
(i) an amino acid sequence as set forth in any one of SEQ ID NOs: 1-9, preferably any one of SEQ ID NOs: 1-4;
(ii) an amino acid sequence with at least 70% sequence identity to an amino acid sequence as set forth in any one of SEQ ID NOs: 1-9, preferably any one of SEQ ID NOs: 1-4; or
(iii) an IL-18 binding fragment of (i) or (ii).

As referred to herein a "fragment" may comprise at least 30, 40, 50, 60, 70, 80, 85, 90, 95, 96, 97, 98 or 99% of the amino acids of the protein from which it is derived. Said fragment may be obtained from central, N-terminal or C-terminal portions of the sequence. Whilst the size of the fragment will depend on the size of the original protein, in some embodiments the fragments may be 1, 2, 3, 4, 5, 6, 7, 8, 9, 10, 11, 12, 13, 14, 15, 20, 30, 40 or more amino acid residues shorter than the sequence from which it is derived, e.g. 1-40, 2-35, 3-30, 4-25 amino acid residues shorter than the sequence from which it is derived.

As referred to herein a "variant" or "derivative" of a sequence is at least 70, 75, 80, 85, 90, 95, 96, 97, 98 or 99% identical to the sequence to which it is compared.

Sequence identity may be determined by, e.g. using the SWISS-PROT protein sequence databank using FASTA pep-cmp with a variable pamfactor, and gap creation penalty set at 12.0 and gap extension penalty set at 4.0, and a window of 2 amino acids. Preferably said comparison is made over the full length of the sequence, but may be made over a smaller window of comparison, e.g. less than 200, 100, 50, 20 or 10 contiguous amino acids.

Preferably such sequence identity related proteins, i.e. variants or derivatives, are functionally equivalent to the proteins which are set forth in the recited SEQ ID NOs. Similarly, the proteins with sequences as set forth in the SEQ ID NOs. may be modified without affecting the sequence of the polypeptide.

Furthermore, "fragments" as described herein are functional equivalents. Preferably these fragments satisfy the identity (relative to a comparable region) conditions mentioned herein.

As referred to herein, to achieve "functional equivalence" the protein fragment and/or variant may show some reduced efficacy in performing the function relative to the parent molecule (i.e. the molecule from which it was derived, e.g. by amino acid substitution), but preferably is as efficient or is more efficient. Thus, functional equivalence refers to a protein that is effective at binding selectively to, and antagonising the function of free IL-18. This may be tested by comparison of the effects of the fragment or variant protein relative to the protein from which it is derived in a qualitative or quantitative manner, e.g. by performing the *in vitro* analyses known in the art and described in WO 2016/139297. Where quantitative results are possible, the fragment or variant is at least 30, 50, 70 or 90% as effective as the parent protein.

Functionally-equivalent proteins which are related to or derived from the parent protein may be obtained by modifying the parent amino acid sequence by single or multiple amino acid substitution, addition and/or deletion (providing they satisfy the above-mentioned sequence identity requirements), but without destroying the molecule's function. Preferably the parent sequence has less than 60 substitutions, additions or deletions, e.g. less than 50, 40, 30, 20, 10, 5, 4, 3 or 2 such modifications. Such proteins may be encoded by "functionally-equivalent nucleic acid molecules" which may be generated by appropriate substitution, addition and/or deletion of one or more bases.

In some embodiments, any substitutions that are present in the variant protein relative to the parent protein may be conservative amino acid substitutions. A conservative amino acid substitution refers to the replacement of an amino acid by another which preserves the physicochemical character of the polypeptide (e.g. D may be replaced by E or vice versa, N by Q, or L or I by V or vice versa). Thus, generally the substituting amino acid has similar properties, e.g. hydrophobicity, hydrophilicity, electronegativity, bulky side chains etc. to the amino acid being replaced. Isomers of the native L-amino acid e.g. D-amino acids may be incorporated.

The mammalian or viral IL-18 binding proteins or antibodies that bind to IL-18 described herein bind selectively to free IL-18. The term "binds selectively" refers to the ability of the protein to bind non-covalently (e.g. by van der Waals forces and/or hydrogen-bonding) to IL-18 with greater affinity and/or specificity than to other components, e.g. other components in the sample (e.g. blood, tissue) in which the IL-18 is present. Thus, the mammalian or viral IL-18 binding protein or antibody may alternatively be viewed as binding specifically and reversibly to free IL-18, under suitable conditions.

The term "free IL-18" refers to IL-18 that is not already bound to its respective IL-18 binding protein or IL-18 receptor.

Binding to IL-18 may be distinguished from binding to other molecules (e.g. peptides or polypeptides) present in a sample. The mammalian or viral IL-18 binding protein or antibody that binds to free IL-18 either does not bind to other molecules (e.g. peptides or polypeptides) present in a sample or does so negligibly or non-detectably that any such non-specific binding, if it occurs, readily may be distinguished from binding to IL-18.

In particular, if the mammalian or viral IL-18 binding protein or antibody that binds to IL-18 binds to molecules other than IL-18, such binding must be transient and the binding affinity must be less than the binding affinity of the IL-18 binding protein or antibody for IL-18. Thus, the binding affinity of a mammalian or viral IL-18 binding protein or an antibody that binds to IL-18 should be at least an order of magnitude more than the other molecules (i.e. non-cognate molecules) present in a sample. Preferably, the binding affinity of the mammalian or viral IL-18 binding protein or antibody that binds to IL-18 should be at least 2, 3, 4, 5, or 6 orders of magnitude more than the binding affinity for other molecules (e.g. peptides or polypeptides).

Thus, selective or specific binding refers to affinity of the mammalian or viral IL-18 binding protein or antibody that binds to IL-18 where the dissociation constant of the mammalian or viral IL-18 binding protein or antibody that binds to IL-18 is less than about 10⁻³ M. In a preferred embodiment the dissociation constant of the mammalian or viral IL-18 binding protein or antibody that binds to IL-18 is less than about 10⁻⁴ M, 10⁻⁵ M, 10⁻⁶ M, 10⁻⁷ M, 10⁻⁸ M or 10⁻⁹ M.

In some embodiments, the IL-18 binding protein fragments and variants described above compete with the parent protein from which they are derived for binding to free IL-18. Thus, the IL-18 binding protein fragments and variants may be viewed as "competing IL-18 binding protein fragments and variants", which term refers to proteins that bind to about, substantially or essentially the same, or even the same, epitope as the parent protein. "Competing IL-18 binding protein fragments and variants" include proteins with overlapping epitope specificities. Competing IL-18 binding protein fragments and variants are thus able to effectively compete with the parent protein for binding to IL-18.

The polypeptides described herein may be an isolated, purified, recombinant or synthesised polypeptides.

The term "polypeptide" is used herein interchangeably with the term "protein". As noted above, the term polypeptide or protein typically includes any amino acid sequence comprising at least 40 consecutive amino acid residues, e.g. at least 50, 60, 70, 80, 90, 100, 150 amino acids, such as 40-1000, 50-900, 60-800, 70-700, 80-600, 90-500, 100-400 amino acids.

In some embodiments, the IL-18 antagonist is an antibody that binds selectively (as defined above) to free IL-18. In some embodiments, the IL-18 antagonist is an antibody that effectively competes with IL-18 binding protein (e.g. any one of the IL-18 binding proteins described herein, preferably one or more (e.g. all) of SEQ ID NOs: 1-4) for binding to free IL-18. Thus, in some embodiments, the antibody binds to about, substantially or essentially the same, or even the same, epitope as IL-18 binding protein, preferably the same epitope as any one of SEQ ID NOs: 1-4.

As will be understood by those in the art, the immunological binding reagents encompassed by the term "antibody" extend to all antibodies and antigen binding fragments thereof, including whole antibodies, dimeric, trimeric and multimeric antibodies; bispecific antibodies; chimeric antibodies; recombinant and engineered antibodies, and fragments thereof.

The term "antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lambda) bodies (scFv-CL fusions); Bispecific T-cell Engager (BiTE) (scFv-scFv tandems to attract T cells); dual variable domain (DVD)-Ig (bispecific format); small immunoprotein (SIP) (kind of minibody); SMIP ("small modular immunopharmaceutical" scFv-Fc dimer; DART (ds-stabilized diabody "Dual Affinity ReTargeting"); small antibody mimetics comprising one or more CDRs and the like.

The techniques for preparing and using various antibody-based constructs and fragments are well known in the art (see Kabat et al., 5th Ed. Public Health Service, National Institutes of Health, Bethesda, MD, 647-669, 1991). Diabodies, in particular, are further described in WO 93/11161; whereas linear antibodies are further described in Zapata et al. (Protein Eng., 8(10):1057-1062, 1995).

Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well-known and described in the art.

The antibodies or antibody fragments can be produced naturally or can be wholly or partially synthetically produced. Thus, the antibody may be from any appropriate source, for example recombinant sources and/or produced in transgenic animals or transgenic plants, or in eggs using the IgY technology. Thus, the antibody molecules can be produced *in vitro* or *in vivo.*

Preferably, the antibody or antibody fragment comprises an antibody light chain variable region (VL) that comprises three CDR domains and an antibody heavy chain variable region (VH) that comprises three CDR domains. Said VL and VH generally form the antigen binding site.

An "Fv" fragment is the minimum antibody fragment that contains a complete antigen-recognition and binding site. This region has a dimer of one heavy chain and one light chain variable domain in tight, non-covalent association. It is in this configuration that the three hypervariable regions (CDRs) of each variable domain interact to define an antigen-binding site on the surface of the VH-VL dimer. Collectively, the six hypervariable regions (CDRs) confer antigen-binding specificity to the antibody.

However, it is well-documented in the art that the presence of three CDRs from the light chain variable domain and three CDRs from the heavy chain variable domain of an antibody is not necessary for antigen binding. Thus, constructs smaller than the above classical antibody fragment are known to be effective.

For example, camelid antibodies have an extensive antigen binding repertoire but are devoid of light chains. Also, results with single domain antibodies comprising VH domains alone or VL domains alone show that these domains can bind to antigen with acceptably high affinities. Thus, three CDRs can effectively bind antigen. Furthermore, it is also known that a single CDR, or two CDRs, can effectively bind antigen.

Notably, it is known that two CDRs can effectively bind antigen, and even confer superior properties than possessed by the parent antibody. For example, it has been shown that two CDRs from a parent antibody (a VH CDR1 and a VL CDR3 region) may retain the antigen recognition properties of the parent molecule but have a superior capacity to penetrate tumours. Joining these CDR domains with an appropriate linker sequence (e.g., from VH FR2) to orientate the CDRs in a manner resembling the native parent antibody produced even better antigen recognition. Therefore, it is known in the art that it is possible to construct antigen binding antibody mimetics comprising two CDR domains (preferably one from a VH domain and one from a VL domain, more preferably, with one of the two CDR domains being a CDR3 domain) orientated by means of an appropriate framework region to maintain the conformation found in the parent antibody.

Thus, although preferred antibodies for use in the invention might comprise six CDR regions (three from a light chain and three from a heavy chain), antibodies with fewer than six CDR regions and as few as one or two CDR regions are encompassed by the invention. In addition, antibodies with CDRs from only the heavy chain or light chain are also contemplated.

In certain embodiments, the antibody or antibody fragment comprises all or a portion of a heavy chain constant region, such as an IgG1, IgG2, IgG3, IgG4, lgA1, IgA2, IgE, IgM or IgD constant region. Preferably, the heavy chain constant region is an IgG1 heavy chain constant region, or a portion thereof. Furthermore, the antibody or antibody fragment can comprise all or a portion of a kappa light chain constant region or a lambda light chain constant region, or a portion thereof. All or part of such constant regions may be produced naturally or may be wholly or partially synthetic. Appropriate sequences for such constant regions are well known and documented in the art. When a full complement of constant regions from the heavy and light chains is included in the antibodies for use in the invention, such antibodies are typically referred to herein as "full length" antibodies or "whole" antibodies.

Antibodies containing an Fc region are preferred for therapeutic uses *in vivo.*

In preferred embodiments, the antibodies for use in the invention are monoclonal antibodies, which may be humanised or human monoclonal antibodies. In this regard, human or humanised antibodies generally have at least three potential advantages for use in human therapy. First, the human immune system should not recognize the antibody as foreign. Second, the half-life in the human circulation will be similar to naturally occurring human antibodies, allowing smaller and less frequent doses to be given. Third, because the effector portion is human, it will interact better with the other parts of the human immune system.

Non-human antibodies may be "humanised" in known ways, for example by inserting the CDR regions of said non-human antibodies into the framework of human antibodies. Humanised antibodies can be made using the techniques and approaches described in Verhoeyen et al (1988) Science, 239, 1534-1536, and in Kettleborough et al, (1991) Protein Engineering, I4(7), 773-783. In some instances, Fv framework residues of the human immunoglobulin are replaced by corresponding non-human residues. In general, the humanised antibody will contain variable domains in which all or most of the CDR regions correspond to those of a non-human immunoglobulin, and framework regions which are substantially or completely those of a human immunoglobulin consensus sequence.

Completely human antibodies may be produced using recombinant technologies. Typically large libraries comprising billions of different antibodies are used. In contrast to the previous technologies employing chimerisation or humanisation of e.g. murine antibodies this technology does not rely on immunisation of animals to generate the specific antibody. Instead the recombinant libraries comprise a huge number of pre-made antibody variants wherein it is likely that the library will have at least one antibody specific for any antigen. Thus, in the context of the present invention, a competing antibody having the desired binding characteristics can be identified using such libraries. In order to find the good binder in a library in an efficient manner, various systems where phenotype, i.e. the antibody or antibody fragment is linked to its genotype, i.e. the encoding gene, has been devised. The most commonly used such system is the so-called phage display system where antibody fragments are expressed, displayed, as fusions with phage coat proteins on the surface of filamentous phage particles, while simultaneously carrying the genetic information encoding the displayed molecule (McCafferty et al, 1990, Nature 348: 552-554). Phage displaying antibody fragments specific for a particular antigen may be selected through binding to the antigen in question. Isolated phage may then be amplified and the gene encoding the selected antibody variable domains may optionally be transferred to other antibody formats, such as e.g. full-length immunoglobulin, and expressed in high amounts using appropriate vectors and host cells well known in the art. Alternatively, the "human" antibodies can be made by immunising transgenic mice which contain, in essence, human immunoglobulin genes (Vaughan et al (1998) Nature Biotechnol. 16, 535-539).

The "human" and "humanised" antibodies for use in the invention may include amino acid residues not encoded by human sequences, e.g., mutations introduced by random or site directed mutations *in vitro,* for example mutations introduced by *in vitro* cloning or PCR. Particular examples of such mutations are mutations that involve conservative substitutions or other mutations (non-conservative substitutions, additions and/or deletions) in a small number of residues of the antibody, e.g., in up to 5, 4, 3, 2 or 1 of the residues of the antibody, preferably e.g., in up to 5, 4, 3, 2 or 1 of the residues making up one or more of the CDRs of the antibody. Certain examples of such "human" and "humanised" antibodies include antibodies and variable regions that have been subjected to standard modification techniques to reduce the amount of potentially immunogenic sites.

Thus, the "human" and "humanised" antibodies for use in the invention include sequences derived from and related to sequences found in humans, but which may not naturally exist within the human antibody germline repertoire *in vivo.* In addition, the human and humanised antibodies for use in the present invention include proteins comprising human consensus sequences identified from human sequences, or sequences substantially homologous to human sequences.

In addition, the human and humanised antibodies for use in the present invention are not limited to combinations of VH, VL, CDR or FR regions that are themselves found in combination in human antibody molecules. Thus, the human and humanised antibodies for use in the invention can include or correspond to combinations of such regions that do not necessarily exist naturally in humans.

In some embodiments, the human antibodies may be fully human antibodies. "Fully human" antibodies, as used herein, are antibodies comprising "human" variable region domains and/or CDRs, as defined above, without substantial non-human antibody sequences or without any non-human antibody sequences. For example, antibodies comprising human variable region domains and/or CDRs "without substantial non-human antibody sequences" are antibodies, domains and/or CDRs in which only up to 5, 4, 3, 2 or 1 amino acids are amino acids that are not encoded by human antibody sequences. Thus, "fully human" antibodies are distinguished from "humanised" antibodies, which are based on substantially non-human variable region domains, e.g., mouse variable region domains, in which certain amino acids have been changed to better correspond with the amino acids typically present in human antibodies.

The "fully human" antibodies for use in the invention may be human variable region domains and/or CDRs without any other substantial antibody sequences, such as being single chain antibodies. Alternatively, the "fully human" antibodies for use in the invention may be human variable region domains and/or CDRs integral with or operatively attached to one or more human antibody constant regions. Certain preferred fully human antibodies are IgG antibodies with the full complement of IgG constant regions.

In other embodiments, "human" antibodies for use in the invention will be part-human chimeric antibodies. "Part-human chimeric" antibodies, as used herein, are antibodies comprising "human" variable region domains and/or CDRs operatively attached to, or grafted onto, a constant region of a non-human species, such as rat or mouse. Such part-human chimeric antibodies may be used, for example, in pre-clinical studies, wherein the constant region will preferably be of the same species of animal used in the pre-clinical testing. These part-human chimeric antibodies may also be used, for example, in *ex vivo* diagnostics (e.g. in the method for selecting a subject for treatment described above), wherein the constant region of the non-human species may provide additional options for antibody detection.

The IL-18 antagonist may be provided in pharmaceutical composition, which may be formulated according to any of the conventional methods known in the art and widely described in the literature. Thus, IL-18 antagonist may be incorporated, optionally together with other active substances, with one or more conventional carriers, diluents and/or excipients.

The pharmaceutical composition described herein may be administered systemically or locally to the subject using any suitable means and the route of administration will depend on formulation of the pharmaceutical composition.

In some embodiments, systemic administration may be particularly useful. "Systemic administration" includes any form administration in which the agent (i.e. IL-18 antagonist) is administered to the body resulting in the whole body receiving the administered agent. Conveniently, systemic administration may be via enteral delivery (e.g. oral) or parenteral delivery (e.g. intravenous, intramuscular, subcutaneous, intratracheal, endotracheal, inhalation).

"Local administration" refers to administration of the agent at the primary site of infection (e.g. the respiratory tract) or in the local vicinity of the primary site of infection, e.g. via inhalation. However, it will be evident that some forms of local administration may result in the whole body receiving the administered agent (e.g. inhalation). Thus, in some embodiments, the agent may be administered to provide an initial local effect and subsequent systemic effect.

Reference to "systemic administration" includes intra-articular, intravenous, intraperitoneal, and subcutaneous injection, infusion, as well as administration via oral, rectal and nasal routes, or via inhalation. Administration by subcutaneous injection or via inhalation is particularly preferred.

Thus, in some embodiments, the IL-18 antagonist may be provided and/or formulated for intranasal, buccal, oral, transmucosal, intratracheal, intravenous, subcutaneous, intraurinary tract, intrarectal, intravaginal, sublingual, intrabronchial, intrapulmonary, transdermal or intramuscular administration. In some embodiments, the IL-18 antagonist (i.e. pharmaceutical composition) is administered by broncho-pulmonary administration.

The pharmaceutical composition may be provided as a liquid, liquid spray, microspheres, semisolid, gel, or powder for transmucosal administration, e.g. intranasal, buccal, oral transmucosal, intratracheal, intraurinary tract, intravaginal, sublingual, intrabronchial, intrapulmonary and/or transdermal administration. Further, the composition may be in a solid dosage form for buccal, oral transmucosal and/or sublingual administration. Intranasal, buccal, oral intratracheal, intraurinary tract, intravaginal, transmucosal and sublingual administrations lead to the disintegration of the composition as described herein in an oral cavity at body temperature and optionally may adhere to the body tissue of the oral cavity. Additionally, the composition as disclosed herein further may include one or more excipient, diluent, binder, lubricant, glidant, disintegrant, desensitizing agent, emulsifier, mucosal adhesive, solubilizer, suspension agent, viscosity modifier, ionic tonicity agent, buffer, carrier, surfactant, flavor, or mixture thereof.

In some embodiments, the composition is formulated as a parenteral, intravenous, tablet, pill, bioadhesive patch, drops, sponge, film, lozenge, hard candy, wafer, sphere, lollipop, disc-shaped structure, suppository or spray. Transmucosal administration is generally rapid because of the rich vascular supply to the mucosa and the lack of a stratum corneum in the epidermis. Such drug transport typically provides a rapid rise in blood concentrations, and similarly avoids the enterohepatic circulation and immediate destruction by gastric acid or partial first-pass effects of gut wall and hepatic metabolism. Drugs typically need to have prolonged exposure to a mucosal surface for significant drug absorption to occur.

Transmucosal routes can also be more effective than the oral route in that these routes can provide for relatively faster absorption and onset of therapeutic action. Further, the transmucosal routes can be preferred for use in treating patients who have difficulty in swallowing tablets, capsules, or other oral solids, or those who have disease-compromised intestinal absorption.

In either of the intranasal or buccal routes, drug absorption can be delayed or prolonged. However, the sublingual route can provide a rapid onset of action where uptake may be almost as rapid as if an intravenous bolus were administered because of the high permeability of the rich blood supply.

The intranasal compositions can be administered by any appropriate method according to their form. A composition including microspheres or a powder can be administered using a nasal insufflator device. Examples of these devices are well-known to those of skill in the art, and include commercial powder systems such as Fisons Lomudal System. An insufflator produces a finely divided cloud of the dry powder or microspheres. The insufflator is preferably provided with a mechanism to ensure administration of a substantially fixed amount of the composition. The powder or microspheres can be used directly with an insufflator, which is provided with a bottle or container for the powder or microspheres. Alternatively, the powder or microspheres can be filled into a capsule such as a gelatin capsule, or other single dose device adapted for nasal administration. The insufflator preferably has a mechanism to break open the capsule or other device. Further, the composition can provide an initial rapid release of the active ingredient followed by a sustained release of the active ingredient, for example, by providing more than one type of microsphere or powder. Further, alternative methods suitable for administering a composition to the nasal cavity are well-known by the person of ordinary skill in the art. Any suitable method may be used. For a more detailed description of suitable methods reference is made to EP2112923, EP1635783, and EP1648406. In one embodiment of the present invention, the pharmaceutical composition may be administered intranasally, i.e. by inhalation and, thus, may be formulated in a form suitable for intranasal administration, i.e. as an aerosol, dry powder formulation or a liquid preparation.

Examples of suitable pharmaceutical carriers, excipients and/or diluents are well-known in the art and include, but are not limited to, a gum, a starch (e.g. corn starch, pregelatinized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, or mixtures thereof.

Pharmaceutically acceptable carriers for liquid formulations are aqueous or non-aqueous solutions, suspensions, emulsions or oils. Examples of non-aqueous solvents are propylene glycol, polyethylene glycol, and injectable organic esters such as ethyl oleate. Examples of oils are those of animal, vegetable, or synthetic origin, for example, peanut oil, soybean oil, olive oil, sunflower oil, fish-liver oil, another marine oil, or a lipid from milk or eggs.

The present invention also relates to transpulmonary administration by inhalation of the pharmaceutical composition as dry powder, gaseous or volatile formulations into systemic circulation via the respiratory tract. Absorption is virtually as rapid as the formulation can be delivered into the alveoli of the lungs, since the alveolar and vascular epithelial membranes are quite permeable, blood flow is abundant and there is a very large surface for adsorption. For instance, aerosols may be delivered from pressure-packaged, metered-dose inhalers (MDIs).

The pharmaceutical composition will generally be administered in a mixture with a suitable pharmaceutical excipient, diluent or carrier selected with regard to the chosen means of inhalation and standard pharmaceutical practice.

In some embodiments, the IL-18 antagonist is provided as a dry powder composition, optionally together with at least one particulate pharmaceutically acceptable carrier, which may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. In a representative embodiment, the carrier is lactose, for example lactose monohydrate or anhydrous lactose.

In some embodiments, the dry powder may be contained as unit doses in capsules of, for example, gelatin or plastic, or in blisters (e.g. of aluminium or plastic), for use in a dry powder inhalation device, which may be a single dose or multiple dose device, preferably in dosage units together with the carrier in amounts to bring the total weight of powder per capsule to from 5 mg to 50 mg. Alternatively, the dry powder may be contained in a reservoir in a multi-dose dry powder inhalation (MDDPI) device adapted to deliver.

Aqueous carriers include water, alcoholic/aqueous solutions, emulsions or suspensions, including saline and buffered media such as phosphate buffered saline solutions, water, emulsions, such as oil/water emulsions, various types of wetting agents, sterile solutions etc. Compositions comprising such carriers can be formulated by well-known conventional methods. Suitable carriers may comprise any material which, when combined with the IL-18 antagonist, retains the biological activity.

Preparations for transmucosal administration may include sterile aqueous or non-aqueous solutions, suspensions, dry powder formulations and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Transmucosal vehicles may include sodium chloride solution, Ringer's dextrose, dextrose and sodium chloride, lactated Ringer's, or fixed oils. Preservatives and other additives may also be present including, for example, antimicrobials, anti-oxidants, chelating agents, and inert gases and the like. In addition, the pharmaceutical composition may comprise proteinaceous carriers, like, e.g., serum albumin or immunoglobulin, preferably of human origin.

In some embodiments, the pharmaceutical composition may also be administered as a controlled-release composition, i.e. a composition in which the active ingredient is released over a period of time after administration. Controlled- or sustained-release compositions include formulation in lipophilic depots (e.g. fatty acids, waxes, oils). In another embodiment, the composition is an immediate-release composition, i.e. a composition in which all the active ingredient is released immediately after administration. Further examples for suitable formulations are provided in WO 2006/085983. For example, the pharmaceutical composition may be provided as liposomal formulations. The technology for forming liposomal suspensions is well-known in the art. The lipid layer employed can be of any conventional composition and can either contain cholesterol or can be cholesterol-free. The liposomes can be reduced in size, as through the use of standard sonication and homogenization techniques. Liposomal formulations containing the pharmaceutical composition can be lyophilized to produce a lyophilizate which can be reconstituted with a pharmaceutically acceptable carrier, such as water, to regenerate a liposomal suspension.

In some embodiments, the pharmaceutical composition is a "ready to use" formulation that contains the IL-18 antagonist in dissolved or solubilized form and is intended to be used as such or upon further dilution in pharmaceutically acceptable (e.g. intravenous) diluents. However, in some embodiments, the pharmaceutical composition may be provided in a solid form, e.g. as a lyophilizate, to be dissolved in a suitable solvent to provide a liquid formulation.

In some embodiments, the IL-18 antagonist may be in the form of a salt, i.e. a pharmaceutically acceptable salt. For instance, the IL-18 antagonist may be in the form of an acidic or basic salt. In some embodiments, the IL-18 antagonist is in a neutral salt form.

Pharmaceutically acceptable salts include pharmaceutical acceptable base addition salts and acid addition salts, for example, metal salts, such as alkali and alkaline earth metal salts, ammonium salts, organic amine addition salts, and amino acid addition salts, and sulfonate salts. Acid addition salts include inorganic acid addition salts such as hydrochloride, sulfate and phosphate, and organic acid addition salts such as alkyl sulfonate, arylsulfonate, acetate, maleate, fumarate, tartrate, citrate and lactate. Examples of metal salts are alkali metal salts, such as lithium salt, sodium salt and potassium salt, alkaline earth metal salts such as magnesium salt and calcium salt, aluminum salt, and zinc salt. Examples of ammonium salts are ammonium salt and tetramethylammonium salt. Examples of organic amine addition salts are salts with morpholine and piperidine. Examples of amino acid addition salts are salts with glycine, phenylalanine, glutamic acid and lysine. Sulfonate salts include mesylate, tosylat and benzene sulfonic acid salts.

"Pharmaceutically acceptable" as referred to herein refers to ingredients that are compatible with other ingredients used in the uses of the invention as well as physiologically acceptable to the recipient.

The terms "effective amount" and "therapeutically effective amount" as used herein mean a sufficient amount of an IL-18 antagonist that provides the desired therapeutic or physiological effect or outcome by inhibiting the activity of IL-18 (as defined above). In addition, the effect may be an amelioration of the symptoms of the viral respiratory infection (e.g. COVID-19), related conditions and/or complications arising from same or manifestations thereof. Undesirable effects, e.g. side effects, may sometimes manifest along with the desired therapeutic effect; hence, a practitioner balances the potential benefits against the potential risks in determining what is an appropriate "effective amount".

The exact amount of IL-18 antagonist required will vary from subject to subject, depending on the numerous parameters such as age and general condition of the subject, mode of administration, weight, body surface area, sex, other drugs being administered concurrently and the like. Thus, it may not be possible to specify an exact "effective amount". However, an appropriate "effective amount" in any individual case may be determined by one of ordinary skill in the art using routine experimentation.

Advantageously, the amount of free IL-18 in the subject may be determined using assays, e.g. immunoassays, that are routine in the art as described above. The amount of free IL-18 may be used to determine the amount of IL-18 antagonist to be administered to the subject.

Thus, the IL-18 antagonist can be administered to the subject at any suitable dose.

The effective amount includes from about 10µg/kg body weight to about 20mg/kg body weight of antagonist such as 10, 20, 30, 40, 50, 60, 70, 80, 90, 100µg/kg body weight, 100, 200, 300, 400, 500, 600, 700, 800, 900, 1000µg/kg body weight or 2, 3, 4, 5, 6, 7, 8, 9, 10, 11, 12, 13, 14, 15, 16, 17, 18, 19 or 20mg/kg body weight.

In some embodiments, the treatment described herein may utilize a combination of IL-18 antagonists, e.g. an IL-18 binding protein and an antibody to IL-18 or IL-18R. The amounts described above may be used for each antagonist agent in such combination therapies.

BSA (Body surface area) may be calculated, for example, using the Mosteller formula (√([height(cm) × weight(kg)]/3600)). Where necessary this may be converted to mg/kg by using a conversion factor for an average adult of 0.025mg/kg = 1 mg/m² BSA.

Thus, the IL-18 antagonist may be administered as an effective amount of about 0.4 mg/m² BSA to about 80 mg/m² BSA of antagonist such as about 0.4, 0.8, 1.2, 1.6, 2.0, 2.4, 2.8, 3.2, 3.6, 4.0 mg/m² BSA, 4, 8, 12, 16, 20, 24, 28, 32, 36, 40 mg/m² BSA or 80, 120, 160, 200, 240, 280, 320, 360, 400, 440, 480, 520, 560, 600, 640, 680, 720, 760 or 800 mg/m² BSA.

The dosage regimen of the IL-18 antagonist will be determined by the attending physician and clinical factors. As is well known in the medical arts, dosages for any one subject depend upon many factors, including the subject's size, body surface area, age, the particular compound to be administered, sex, time and route of administration, general health, and other drugs being administered concurrently.

By way of example, in some embodiments of the invention the IL-18 antagonist is administered daily or every 2-3 days. In some embodiments, the IL-18 antagonist may be administered for at least three days, e.g. for 3, 4, 5, 6, 7, 8, 9 10 or more days (e.g. 20, 25, 30 or more days). This administration may be in a single cycle or in total in multiple cycles.

As referred to herein a "cycle" is a time period over which a particular treatment regime is applied and is generally repeated to provide cyclical treatment. The treatment in each cycle may be the same or different (e.g. different dosages, timings etc. may be used). In some embodiments, a cycle may be from 3-6 or 3-12 days in length, e.g. a 3, 4, 6, 9 or 12 day cycle. In some embodiments, a cycle may be about 1-6 weeks, i.e. daily administration for about 1-6, e.g. 1-4 or 1-3 weeks, such as about 1 or 2 weeks. In some embodiments, the cycle is repeated at least once. Thus, multiple cycles may be used, e.g. at least 2, 3, 4 or 5 cycles, e.g. 6, 7, 8, 9 or 10 (e.g. 10, 20, 30 or more) cycles.

In some embodiments, treatment cycles may be delimited by a break in treatment, e.g. a period without daily administration of the IL-18 antagonist. In some embodiments, the period between cycles is at least one day, e.g. 2, 3, 4 or more days. In some embodiments, the period between cycles is at least one week, e.g. 2, 3, 4 or more weeks.

However, in some embodiments, the second or subsequent treatment cycle may immediately follow the first or previous cycle. For instance, if the third daily dose of the first cycle was administered on day 3 ± 1 day, the first daily dose of the second cycle may be administered on day 4 ± 1 day.

In some embodiments of the invention, the patient may be subjected to other treatments prior to, contemporaneously with, or after the treatments of the present invention. For instance, in some embodiments, the patient may be treated with other procedures for the treatment of symptoms associated with the viral respiratory infection, e.g. assisted ventilation according to procedures known in the art.

In some embodiments, the IL-18 antagonist may be administered in combination with other therapeutic agents for the treatment of symptoms associated with the viral respiratory disease or other underlying condition, e.g. metabolic syndrome, CAD, hypertension etc.

Thus, in some embodiments, the pharmaceutical composition containing the IL-18 antagonist may contain one or more additional therapeutic agents or may be for administration with one or more additional therapeutic agents.

In some embodiments, the pharmaceutical composition containing the IL-18 antagonist may contain or be administered with a further therapeutic agent useful in treating a viral infection, i.e. an antiviral agent. For instance, oseltamivir and zanamivir are effective for treating influenza. Ribavirin, a guanosine analog that inhibits replication of many RNA and DNA viruses, may find utility in the treatment of patients with lower respiratory tract infection due to RSV. Palivizumab, a monoclonal antibody to RSV fusion protein, may be to treat RSV infection. A suitable antiviral agent may be selected based on the viral infection.

In some embodiments, the pharmaceutical composition containing the IL-18 antagonist may contain or be administered with a further therapeutic agent useful in treating a symptom of the infection, such as shortness of breath. For instance, the further therapeutic agent may be an inhaled bronchodilator and/or corticosteroid. For instance, the pharmaceutical composition containing the IL-18 antagonist may contain or be administered with a long-acting beta-adrenoceptor agonist (LABA), such as formoterol, and/or a steroid, such as beclomethasone.

The other therapeutic agents may be part of the same composition already comprising the IL-18 antagonist, in the form of a mixture, wherein the IL-18 antagonist and the other therapeutic agent are intermixed in or with the same pharmaceutically acceptable solvent and/or carrier or may be provided separately as part of a separate compositions, which may be offered separately or together in form of a kit of parts.

Thus, the IL-18 antagonist may be administered concomitantly with the other therapeutic agent separately, simultaneously or sequentially. For example, the IL-18 antagonist may be administered simultaneously with a first additional therapeutic agent or sequentially after or before administration of said first additional therapeutic agent. If the treatment regimen or schedule utilizes more than one additional therapeutic agent, the various agents may be partially administered simultaneously, partially sequentially in various combinations.

Thus, disclosed herein is an IL-18 antagonist in a combined product with another therapeutic agent (e.g. an antiviral agent, bronchodilator, steroid etc.) for separate, simultaneous or sequential administration for use in treating a viral respiratory infection in a subject having metabolic syndrome, coronary artery disease, hypertension, atherosclerosis, diabetes or a combination thereof, wherein the IL-18 antagonist is:
(a) a mammalian or viral IL-18 binding protein that binds free IL-18; or
(b) an antibody that binds specifically to free IL-18.

The therapeutic agents for use in combination with the IL-18 antagonist may be provided in pharmaceutical compositions as defined above and may be administered as defined above. Thus, the compositions comprising additional therapeutic agents may comprise pharmaceutically acceptable excipients, solvents and diluents suitable for such formulations.

The skilled person will be aware of suitable dosage ranges for any given additional therapeutic agent. In preferred embodiments, the additional therapeutic agent is present in the pharmaceutical composition, or administered to the subject, in its typical dose range.

The invention will now be further described with reference to the following non-limiting Examples and Figures in which:
Figure 1 shows *age distribution across severity of disease (worst PF ratio).*
Figure 2 shows proportions of patients by comorbidity score (0 = no diabetes or hypertension; 1 = hypertension or diabetes; 2 = diabetes and hypertension) with mild (Worst PFR > 300), moderate (300 < Worst PFR < 150) and severe (Worst PFR < 150) COVID-19 disease.
Figure 3 shows the relationship between 60-day mortality (Y = died; N = survived) and worst PF Ratio during recorded during inpatient stay category.
Figure 4 shows the relationship between Hypertension and Day-60 Mortality across different severity levels of COVID-19 disease, as determined by Worst PFR recorded. Fisher's Exact Test used to examine significance of difference between those who died and those who survived in proportions of patients with comorbidity of hypertension.
Figure 5 shows BMI per patient disease severity (worst PFR). The most severe category have significantly higher BMI than those in the moderate category (p=0.023) and those in the mildest category (p=2.161×10⁻⁰⁶).
Figure 6 shows patient BMI split according to age range (threshold: 60 years of age) against disease severity (worst PFR).
Figure 7 shows Free IL18 levels by oxygen delivery method as marker of disease severity. Patients intubated and on HFNO show statistically significant higher levels of Free IL18 as compared to patients with requiring minimal or no oxygenation, but still hospitalised with COVID-19.

### EXAMPLES

### Example 1 - An immunological model of COVID-19

Infection with SARS-CoV2 has been described as having two phases,¹ with the majority of individuals experiencing only the first phase. The first phase consists of a flu-like illness, with sufferers describing intermittent fevers, lethargy and a new onset continuous cough. The second phase, starting at around 5-7 days of symptoms is characterised by sudden onset shortness of breath that becomes progressively worse. It is at this stage that patients usually present in the hospital. Those patients that require invasive ventilation on Intensive Care have been noted as strikingly similar, not only in demographic characteristics, but also in clinical presentation. In our hospital in East Surrey, UK, patients requiring Intensive Care support are typically middle aged to elderly males, with one or more metabolic syndrome conditions. Ethnicity has also been noted as a common feature, with a disproportionate number being Black and Ethnic Minorities (BAME)². Biochemical presentation includes lymphopenia, hyperferritinemia and unrecordable high CRP levels, with clinical features consisting of spiking fevers, hypothermia and acute respiratory distress syndrome (ARDS). Some patients can develop liver failure, kidney failure or encephalopathy, with many patients showing uncontrolled hypertension.

The above observations have been widely recognised, but the underlying reasons for them have not. The model of COVID-19 immunopathology described below is based on major streams of COVID-19 specific data, histopathological autopsy analysis, transcriptomic and immune analysis of bronchoalveolar lavage fluid, and peripheral blood flow cytometric analysis. This unique model of COVID-19 immunopathology has resulted in the surprising determination that blockade of IL-18 activity will find utility in the treatment of subjects with viral respiratory infections, such as COVID-19, particularly in subjects with an acquired innate immune deficiency (i.e. presenting as a deficiency in IL-18BP, NK cells and/or functional NK cells) such as in subjects with metabolic syndrome, coronary artery disease, hypertension, atherosclerosis, diabetes or a combination thereof.

### Summary of the major streams of COVID-19 specific data

Histopathological analysis across a series of studies^{3,4,5,6,7,8} has revealed similar and dissimilar features. Similar features include the presence of diffuse alveolar disease, alveolar oedema and proteinaceous exudates, with the formation of hyaline membranes, all indicative of ARDS. Divergence is found on the degree of inflammatory cell tissue infiltrate, with some papers finding none^{1,5} as compared to low to moderate amounts^{2,3,4} though the finding of mononuclear inflammatory cells in alveolar air spaces with or without multinucleated syncytial cells and desquamated, enlarged type II pneumocytes is reported reliably across all studies. Particularly interesting was the mention in several studies of the predominance of CD4+ T lymphocytic^{4,6} infiltrates as compared to a relative paucity of scattered CD8+ cells⁴. In addition, autopsy analysis of spleens from 10 patients with COVID-19 have demonstrated lymphocytic depletion with signs of necrosis and degeneration, indicating a potential cytophatic effect of the virus on lymphocytes as a mechanism of viral evasion.⁹

Bronchoalveolar lavage fluid (BALF) immune analysis¹⁰ of 6 COVID-19 positive patients (3 with severe disease; 3 with mild disease) demonstrates two major findings. The first relates to a massive clonal expansion of macrophages in severe disease as compared to mild disease and healthy controls, along with a change in the macrophage population towards pro-fibrotic and alveolar macrophage types, away from the monocyte-derived type. The second relates to a preponderance of CD8+ T-cells in mild COVID-19 disease as compared to severe disease, with greater activation of CD4+ T-cells (T-regulatory cells, proliferating cells and CCR7+ cells) in severe disease. Of note, more than 50% of CD8+ T cells in mild COVID-19 disease were expanded clones, likely representing SARS-CoV2 specific cytotoxic T-cells, due to repeated antigenic presentation. The amplification index of these clonal CD8+ T cells was significantly higher in all three mild COVID-19 cases, as compared to the severe cases, from which the authors surmised that early and rapid specific CD8+ T cell expansion was key in limiting viral replication and activity. Transcriptomic analysis¹¹ of BALF and peripheral blood mononuclear cells (PBMCs) through RNA-seq processing, to identify up-regulated genes specific to COVID-19 infection, revealed a "cytokine storm" type picture in which, contrary to the autopsy analysis of spleens described above, viral readings were high in BALF but absent in PBMCs. Instead, PBMCs showed evidence of significant upregulation in the p53 apoptotic signalling pathway gene. Taken together, these findings may indicate that programmed cell death, may also be a key cause of clinically observed lymphopenia, predictive of disease severity.¹²

Flow cytometry analysis¹³ of peripheral blood cells in SARS-CoV2 positive patients found that at hospital admission, mild disease was characterised by decreased CD8+ T cell numbers, as compared to healthy controls, with preserved natural killer (NK) cell numbers, while severe disease was characterised by a depletion of both. In addition, functional failure of NK cells was also seen, with IFN-γ and Granzyme B release significantly lower in COVID-19 patients as compared to healthy controls. Longitudinal flow-cytometry analysis, correlating cellular analysis with clinical outcomes,¹⁴ found depletion in CD8+ T-cells in severe as opposed to mild cases, at time points before day 3, and between days 7-9 of the disease, reached the significance threshold, while CD4+, CD3+ and NK cell numbers were non-significantly decreased throughout the analysis period. This was confirmed by more extended analysis,¹⁵ which found that day 10 of the disease is a key time-point, separating patients into three groups: mild, moderate and severe, according to both degree of lymphopenia and clinical severity. Patients who died of severe disease showed lymphopenia below 5% persistently, throughout their disease after day 13, while severe cases who recovered never showed a fall in lymphocyte count below 9%. Analysis of the particular lymphocytic subsets beyond early infection has demonstrated a Th1 response^{16,17}. CD4+ Th1 cells in patients with severe disease express aberrant co-expression of GM-CSF, IL-6 and IFN-γ, with CD8+ T-cells also demonstrating higher levels of GM-CSF expression. Peripheral blood monocytes in such patients co-expressed CD14 and CD16, the signature of a high inflammatory state, indicating their activation by the cytokine milieu induced by Th1 activation. Of particular note, these monocytes were capable of secreting both GM-CSF and IL-6 too. Thus, while CD4+ Th1 cells secrete GM-CSF IL-6, attracting peripheral blood mononuclear cells to invade the lung as macrophages, mediating epithelial injury and ARDS, monocytes also secrete GM-CSF and IL-6, to stimulate myelopoiesis and attract more mononuclear cells.

Bringing these streams of evidence together, a relatively consistent picture emerges: severe disease is characterised by depletion and functional exhaustion of NK cells and CD8+ T cells, alongside a subsequent Th1 response, while mild disease is characterised by a pivot towards CD8+ T-cell activation with preserved NK cell numbers and function. Since NK cells and CD8+ T cells are critical for viral clearance, this makes sense. To put these facts in their correct context however, an understanding of the process of virus-induced inflammation is required.

### NK cell Dysfunction Underpins Poor Viral Control

NK cells constitute a first line of defence that even precede the peak of the cytotoxic T-cell response. NK cells are able to directly bind and lyse cells, and are activated to do so through integration of inputs from activating natural killer cell receptors (aNKR) and inhibitory natural killer cell receptors (iNKR), with loss of iNKRs often sufficient to stimulate lysis of a target cell¹⁸. iNKRs consist of the subsets of the major histocompatibility class 1 (MHC-I) human leukocyte antigens (HLA) A, B or C, while aNKRs consist of two major groups: NKG2D receptors, such as UL-16 Binding Proteins (ULBP) and the MHC-I related chain (MIC) proteins, and the natural cytotoxicity receptors (NCRs) NKp30, NKp44, NKp46. NK cell activation in response to a virus is vital, and buys time for the cytotoxic T-cell response as well as the later adaptive response to ramp up.

The importance of NK cells in viral control comes to the fore in Macrophage Activation Syndrome (MAS). MAS is a form of secondary Hemophagocytic lymphohistiocytosis (HLH); primary or "familial" HLH is caused by genetic defects in perforin deployment.¹⁹ Perforin insertion into antigen presenting cells (APCs) are the key means by which NK cells and CD8+ T cells eliminate virally infected cells. Some genetic defects relate to pore formation (PRF1) while others relate to vesicle priming (UNC13D), vesicle fusing (RAB27A), vesicle docking (STX11), or other functions relating to perforin delivery and release. Failure of this key pathway means that NK cells and CD8+ T cells are unable to destroy APCs, despite being continuously challenged by antigens. The consequence of super-antigen presentation is activation of the inflammasome, an understanding of which is key to appreciating this model of IL-18 mediated pathogenesis described herein.

One of the mortality risk factors associated with severe COVID-19 disease has been demonstrated in one retrospective study²⁰ as increasing age. This study also demonstrated significant differences between those who did not survive COVID-19 as compared to those who died, in features of the metabolic syndrome, notably in incidence of hypertension (48% vs 23%) diabetes (31% vs 14%) and coronary artery disease (24% vs 1%). NK cells are impaired both with increasing age and in metabolic syndrome conditions. With increasing age, both the cytotoxic capacity of NK cells and their capability to secrete cytokines, become impaired²¹. In metabolic syndrome conditions, which are characterised by high levels of circulating free fatty acids, NK cells undergo "re-programming", as a result of peroxisome proliferator-activated receptor (PPAR)-driven lipid accumulation, through disruption of mTOR-mediated glycloysis²². This is important because successful switching to mTOR-mediated glycolysis from oxidative phosphorylation is necessary for NK cellular activation, and associated functions such as trafficking of the cytotoxic machinery to the NK cell-target synapse²³, with inhibition of lipid transport into NK cell mitochondria restoring cytotoxic function. This may explain the demographic observation that individuals from an African and Indo-Pakistani Asian background are disproportionately suffering from the severe manifestation of COVID-19, since these groups in particular deposit adipose tissue abdominally, accounting for their greater propensity to diabetes at lower obesity levels than European counterparts²⁴. More diabetogenic fat may result in greater free fatty acid levels in the blood, and subsequent greater accumulation of fatty acids in NK cells. The consequence may be what has already been demonstrated and cited above in severe COVID-19 disease: failure of NK cell proliferation and function.

One particular manner by which NK cell failure may precipitate CD8+ exhaustion and functional depletion, is through the immunomodulatory role NK cells play. In addition to direct antiviral actions, NK cells also play an immunomodulatory role in response to viral infection, by acting to *sustain* CD8+ T cell populations and functions, by preventing rapid burnout. This was demonstrated elegantly in 2012 as a general principle of viral infections, from lymphocytic choriomeningitis virus (LCMV) Arenavirus, Pichinde virus, and Coronavirus mouse hepatitis virus²⁵. This team showed that NK cells directly lyse activated CD4+ cells during viral infection. Since CD4+ costimulation is necessary for an effective CD8+ response, the consequence is a weaker CD8+ response. When the viral dose is high, this is important, since it helps to prolong the response to an acute viral infection, helping to ultimately clear it without fatal immunopathology secondary to over-stimulation of CD4+ and CD8+ T-cells, resulting in functional exhaustion and/or a cytokine storm. When the viral dose is moderate, this action of NK cells is detrimental to the host response, but the viral dose is not high enough to cause fatality. Thus, the NK cell acts to immunomodulate the host innate response, balancing antiviral activity against immunopathology.

Although NK cells are also known to lyse CD8+ T cells directly, especially when the latter become virally infected and downregulate the iNKR interferon I activating receptor (IFNAR),²⁶ this is unlikely to be the main cause of CD8+ T cell depletion in severe COVID-19, not least because severe COVID-19, as demonstrated, is characterised by early NK cell depletion and failure too. It is more likely therefore that failure of NK cell-CD4+ T cell regulation sits at the heart of CD8+ T cell functional exhaustion and depletion in severe COVID-19.

Thus, the result of a deficiency in cytotoxic activity of NK cells due to increasing age or metabolic syndrome conditions is poorer viral control. Viral escape results in repeated antigenic stimulation, and unopposed activation of the inflammasome.

### Inflammasome-NK Cell Interactions Regulate Free IL-18

The inflammasome is a cytosolic multiprotein complex, activated by interferons (IFNs) released from dendritic cells and macrophages upon recognition of bacterial or viral "pathogen-associated molecular patterns" or "danger-associated molecular patterns" released by damaged or dying cells. There are different types of inflammasomes, categorised into pyrin-domain containing sensors (NLRP3, AIM2 and Pyrin) and caspase-activation and recruitment domain (CARD) containing sensors (NLRC4 and NLRP1b)²⁷. Inflammasomes are stimulated by inputs as diverse as ATP, bacterial toxins, viral DNA or RNA, potassium efflux, calcium influx, and even different types of crystals. In addition, osmotic stress in the form of hyperosmolality has also been found to trigger both NLRP3 and NLRC4 inflammasomes²⁸. The inflammasomes are thus activated by a wide variety of inputs representing a diverse array of cellular stress events. Inflammasomes end with cleavage, by active subunits of caspase 1 (p10 and p20), of IL-18 and IL-1β from their pro to active forms, in addition to the insertion of pore-forming gasdermin D (GSDMD), which induces pyroptotic cell death by causing swelling and bursting of the cell. Among the two most well studied inflammasomes are NLRP3 and NLRC4, with mutations in NLRC4 having been shown to generate widespread inflammation through the uncontrolled production of free IL-18, through unopposed activation of toll-like receptor 9 (TLR9).

IFN-γ release from NK cells under the influence of IL-18 constitutes a negative feedback loop, as IFN-γ is likely an essential promoter of IL-18BP transcription in humans, as it is in murine models²⁹. Given that IFN-γ levels are lower or equivalent in severe cases of COVID-19 as compared to moderate or mild cases³⁰, and that, as compared to healthy controls, intracellular IFN-γ levels in NK cells of COVID-19 patients are very significantly collapsed³¹, and that the IL-6/IFN-γ ratio is emerging as a correlative marker of disease severity³², we postulate that IFN-gamma is insufficiently stimulated to trigger release of sufficient IL-18BP in severe form of the disease, when IL-18 is released in much larger quantities in severe disease. The consequence of this would be high free IL-18 levels, even when IL-18 is not excessively released, principally because of a collapse in the production of its binding protein.

### References

1. Chen N, Zhou M, Dong X, Qu J, Gong F, Han Y, Qiu Y, Wang J, Liu Y, Wei Y, Yu T. Epidemiological and clinical characteristics of 99 cases of 2019 novel coronavirus pneumonia in Wuhan, China: a descriptive study. The Lancet. 2020 Feb 15;395(10223):507-13.
2. Pareek M, Bangash MN, Pareek N, Pan D, Sze S, Minhas JS, Hanif W, Khunti K. Ethnicity and COVID-19: an urgent public health research priority. The Lancet. 2020 Apr 21.
3. Tian S, Hu W, Niu L, Liu H, Xu H, Xiao SY. Pulmonary pathology of early phase 2019 novel coronavirus (COVID-19) pneumonia in two patients with lung cancer. Journal of Thoracic Oncology. 2020 Feb 28.
4. Xu, Zhe, Lei Shi, Yijin Wang, Jiyuan Zhang, Lei Huang, Chao Zhang, Shuhong Liu et al. "Pathological findings of COVID-19 associated with acute respiratory distress syndrome." The Lancet respiratory medicine (2020).
5. Zhang H, Zhou P, Wei Y, Yue H, Wang Y, Hu M, Zhang S, Cao T, Yang C, Li M, Guo G. Histopathologic Changes and SARS-CoV-2 Immunostaining in the Lung of a Patient With COVID-19. Annals of Internal Medicine. 2020 Mar 12.
6. Fox SE, Akmatbekov A, Harbert JL, Li G, Brown JQ, Vander Heide RS. Pulmonary and Cardiac Pathology in Covid-19: The First Autopsy Series from New Orleans. medRxiv. 2020 Jan 1.
7. Tian S, Xiong Y, Liu H, Niu L, Guo J, Liao M, Xiao SY. Pathological study of the 2019 novel coronavirus disease (COVID-19) through postmortem core biopsies. Modern Pathology. 2020 Apr 14:1-8.
8. Yao XH, Li TY, He ZC, Ping YF, Liu HW, Yu SC, Mou HM, Wang LH, Zhang HR, Fu WJ, Luo T. A pathological report of three COVID-19 cases by minimally invasive autopsies. Zhonghua bing li xue za zhi= Chinese journal of pathology. 2020 Mar 15;49:E009-.
9. Xu X, Chang XN, Pan HX, Su H, Huang B, Yang M, Luo DJ, Weng MX, Ma L, Nie X. Pathological changes of the spleen in ten patients with new coronavirus infection by minimally invasive autopsies. Zhonghua Bing li xue za zhi= Chinese Journal of Pathology. 2020 Apr 27;49:E014-.
10. Liao M, Liu Y, Yuan J, Wen Y, Xu G, Zhao J, Chen L, Li J, Wang X, Wang F, Liu L. The landscape of lung bronchoalveolar immune cells in COVID-19 revealed by single-cell RNA sequencing. medRxiv. 2020 Jan 1.
11. Xiong Y, Liu Y, Cao L, Wang D, Guo M, Jiang A, Guo D, Hu W, Yang J, Tang Z, Wu H. Transcriptomic characteristics of bronchoalveolar lavage fluid and peripheral blood mononuclear cells in COVID-19 patients. Emerging Microbes & Infections. 2020 Jan 1;9(1):761-70.
12. Tan, Li, Qi Wang, Duanyang Zhang, Jinya Ding, Qianchuan Huang, Yi-Quan Tang, Qiongshu Wang, and Hongming Miao. "Lymphopenia predicts disease severity of COVID-19: a descriptive and predictive study." medRxiv (2020).
13. Zheng M, Gao Y, Wang G, Song G, Liu S, Sun D, Xu Y, Tian Z. Functional exhaustion of antiviral lymphocytes in COVID-19 patients. Cellular & molecular immunology. 2020 Mar 19:1-3.
14. Liu J, Li S, Liu J, Liang B, Wang X, Wang H, Li W, Tong Q, Yi J, Zhao L, Xiong L. Longitudinal characteristics of lymphocyte responses and cytokine profiles in the peripheral blood of SARS-CoV-2 infected patients. EBioMedicine. 2020 Apr 18:102763.
15. Tan L, Wang Q, Zhang D, Ding J, Huang Q, Tang YQ, Wang Q, Miao H. Lymphopenia predicts disease severity of COVID-19: a descriptive and predictive study. Signal transduction and targeted therapy. 2020 Mar 27;5(1):1-3.
16. Weiskopf D, Schmitz KS, Raadsen MP, Grifoni A, Okba NM, Endeman H, van den Akker JP, Molenkamp R, Koopmans MP, van Gorp EC, Haagmans BL. Phenotype of SARS-CoV-2-specific T-cells in COVID-19 patients with acute respiratory distress syndrome. medRxiv. 2020 Jan 1.
17. Zhou, Yonggang, Binqing Fu, Xiaohu Zheng, Dongsheng Wang, Changcheng Zhao, Rui Sun, Zhigang Tian, Xiaoling Xu, and Haiming Wei. "Pathogenic T cells and inflammatory monocytes incite inflammatory storm in severe COVID-19 patients." National Science Review (2020).
18. Tremblay-McLean A, Coenraads S, Kiani Z, Dupuy FP, Bernard NF. Expression of ligands for activating natural killer cell receptors on cell lines commonly used to assess natural killer cell function. BMC immunology. 2019 Dec;20(1):8.
19. Crayne CB, Albeituni S, Nichols KE, Cron RQ. The immunology of macrophage activation syndrome. Frontiers in immunology. 2019;10.
20. Zhou, Fei, Ting Yu, Ronghui Du, Guohui Fan, Ying Liu, Zhibo Liu, Jie Xiang et al. "Clinical course and risk factors for mortality of adult inpatients with COVID-19 in Wuhan, China: a retrospective cohort study." The Lancet (2020).
21. Hazeldine J, Lord JM. The impact of ageing on natural killer cell function and potential consequences for health in older adults. Ageing research reviews. 2013 Sep 1;12(4):1069-78.
22. Michelet X, Dyck L, Hogan A, Loftus RM, Duquette D, Wei K, Beyaz S, Tavakkoli A, Foley C, Donnelly R, O'Farrelly C. Metabolic reprogramming of natural killer cells in obesity limits antitumor responses. Nature immunology. 2018 Dec;19(12):1330-40.
23. Isaacson B, Mandelboim O. Sweet Killers: NK Cells Need Glycolysis to Kill Tumors. Cell metabolism. 2018 Aug 7;28(2):183-4.
24. Tillin T, Sattar N, Godsland IF, Hughes AD, Chaturvedi N, Forouhi NG. Ethnicity-specific obesity cut-points in the development of Type 2 diabetes-a prospective study including three ethnic groups in the United Kingdom. Diabetic Medicine. 2015 Feb;32(2):226-34.
25. Waggoner SN, Cornberg M, Selin LK, Welsh RM. Natural killer cells act as rheostats modulating antiviral T cells. Nature. 2012 Jan;481(7381):394-8.
26. Crouse J, Bedenikovic G, Wiesel M, Ibberson M, Xenarios I, Von Laer D, Kalinke U, Vivier E, Jonjic S, Oxenius A. Type I interferons protect T cells against NK cell attack mediated by the activating receptor NCR1. Immunity. 2014 Jun 19;40(6):961-73.
27. Malik A, Kanneganti TD. Inflammasome activation and assembly at a glance. J Cell Sci. 2017 Dec 1;130(23):3955-63.
28. Ip WE, Medzhitov R. Macrophages monitor tissue osmolarity and induce inflammatory response through NLRP3 and NLRC4 inflammasome activation. Nature communications. 2015 May 11;6(1):1-1.
29. Hurgin V, Novick D, Rubinstein M. The promoter of IL-18 binding protein: activation by an IFN-γ-induced complex of IFN regulatory factor 1 and CCAAT/enhancer binding protein β. Proceedings of the National Academy of Sciences. 2002 Dec 24;99(26):16957-62.
30. Chen G, Wu D, Guo W, Cao Y, Huang D, Wang H, Wang T, Zhang X, Chen H, Yu H, Zhang X. Clinical and immunological features of severe and moderate coronavirus disease 2019. The Journal of clinical investigation. 2020 Apr 13;130(5).
31. Zheng M, Gao Y, Wang G, Song G, Liu S, Sun D, Xu Y, Tian Z. Functional exhaustion of antiviral lymphocytes in COVID-19 patients. Cellular & molecular immunology. 2020 Mar 19:1-3.
32. Lagunas-Rangel FA, Chávez-Valencia V. High IL-6/IFN-γ ratio could be associated with severe disease in COVID-19 patients. Journal of Medical Virology. 2020 Apr 16.

### Example 2 - Assessment of Free IL-18 levels in subjects with COVID-19

The immunological model of COVID-19 discussed in Example 1 points to Free IL18 as playing an important role in the pathogenesis of the severe form of COVID-19 and forming the basis of a new drug target to prevent or treat the severe form of the disease. To investigate this hypothesis, research into the levels of Free IL18 at all levels of disease severity in patients with COVID-19, at East Surrey Hospital, Surrey, UK, between 9th October 2020 and 9th January 2021, was undertaken.

As discussed above, IL18 is regulated by an endogenous ligand: IL18-Binding Protein (IL18BP). IL18BP is constitutively produced by mononuclear cells and is found normally in circulation at a concentration of 2.5ng/ml. IL18BP has a high affinity for IL18 and effectively silences its biological activity. Thus, it is only free-IL18, unbound to its circulation endogenous ligand, that constitutes the biologically active form of this interleukin. This is a critical point for studies that seek to measure IL18; without measuring the binding protein and thus, the free portion of total-IL18, one cannot know the true reality of biologically active IL18 levels.

### Methods

### Overview

Excess blood samples (total 947) were collected longitudinally from routine clinical blood tests of 272 individuals over the age of 18, admitted as inpatients with COVID-19 to East Surrey Hospital, between 9th October 2020 and 9th January 2021.

Comorbidity data (relating to diabetes and hypertension) and Mortality data, as determined by 60-day mortality and worst PF Ratio (Pa02/Fi02) detected during admission, is presented here for all 272 patients. BMI was only obtainable in 146 patients of 272. Free IL18 results are presented from a subset of 211 of the total 947 blood samples, randomly selected from 116 of 272 patients, at every level of disease severity.

### Data Collection

Excess blood of 0.5ml - 1ml was aliquoted from EDTA and Serum samples. Whenever EDTA and Serum samples were available for the same blood-taking event, both were sampled and stored. Centrifugation of samples occurred after at least 30 minutes standing. Aliquoting of samples post centrifugation was followed by immediate labelling and storage of samples at -75 degrees Celsius. No thawing periods occurred between aliquoting and analysis of any sample.

In addition to sample analysis, each patient's age, sex, BMI and comorbidity profile as relating to diabetes and hypertension, were recorded, with a comorbidity score formulated (0 = no diabetes or hypertension; 1 = diabetes or hypertension; 2 = diabetes and hypertension). Biochemical features of the disease, including lymphocyte count, percentage lymphopaenia, neutrophil:lymphocyte ratio, CRP level and Ferritin level for each individual blood-taking event were recorded. The following oxygenation parameters at each blood taking event were recorded to assess for severity of disease: PF ratio (Pa02/Fi02), as per the Berlin criteria for severity in acute respiratory distress syndrome (ARDS), and method of oxygen delivery (spontaneous; nasal cannula; face-mask; high-flow nasal oxygen {HFNO}; continuous or bi-level positive airway pressure {CPAP/BIPAP}; intubation). When Pa02 was not available, it was calculated through oxygen saturation (Sa02) records, as per equations given in the literature (Sa02/Fi02=29.6+1.09{Pa02/Fi02}). For patients intubated on intensive care, additional note was made of: number of supported organs (of: vasopressor use, respiratory support or renal-replacement therapy), renal-replacement therapy requirement, ventilation parameters (peak inspiratory airway pressure {Pinsp} and positive end-expiratory pressure {PEEP}), number of ventilated days and number of days in intensive care.

### Sample Analysis

Samples were thawed at room temperature before enzyme-linked immunosorbent assay (ELISA) analysis. Total IL18 was analysed from EDTA samples (Human total IL-18/IL-1F4 Quantikine ELISA kit, R&D Systems, Minneapolis, MN, USA); IL18BP from EDTA samples (Human IL-18 BPa Quantikine ELISA kit, R&D Systems, Minneapolis, MN, USA) and IL18-BP-Complex from Serum samples (Human IL-18/IL-18 BPa Complex DuoSet ELISA, R&D Systems, Minneapolis, MN, USA; DuoSet ELISA Ancillary Reagent Kit 2, R&D Systems, Minneapolis, MN, USA). EDTA and Serum samples analysed were always from the same blood-taking event. ELISA procedures were undertaken in accordance with the ELISA protocols set out in the product datasheets of each kit.

### Statistical Analysis

Levels of Total IL18, IL18BP and IL18-BP-Complex were measured using the above stated methods, and Free IL18 levels were calculated as per the law of Mass Action, detailed in Novick et al. (Cytokine. 2001 Jun 1;14(6):334-42), using a Kd of 0.05. Statistical analysis was undertaken using R.

### Results

### Demographics and Comorbidity

Analysis of the demography of all 272 patients revealed interesting findings.

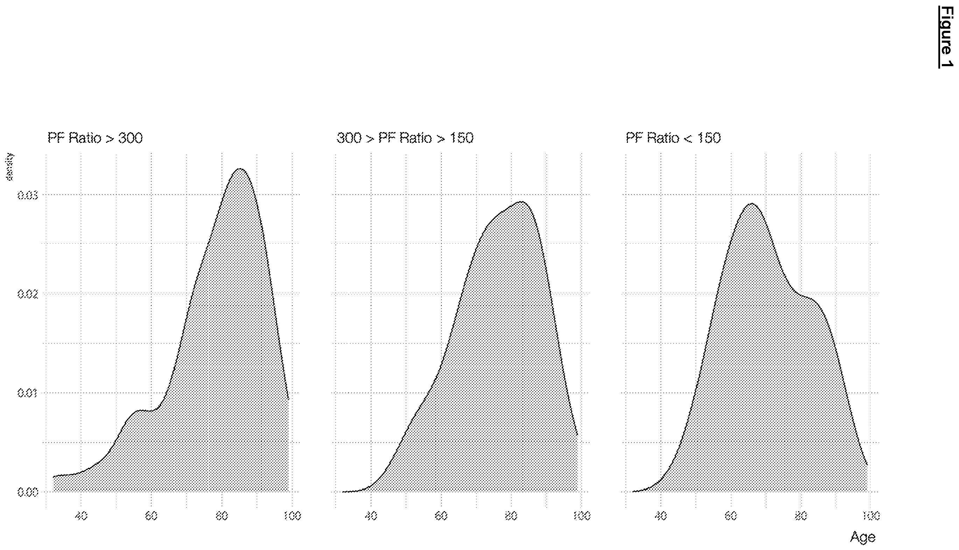

Firstly, age was relatively evenly distributed between all categories of disease severity (Fig.1). No significant difference was seen in age of males vs females, and so is not shown.

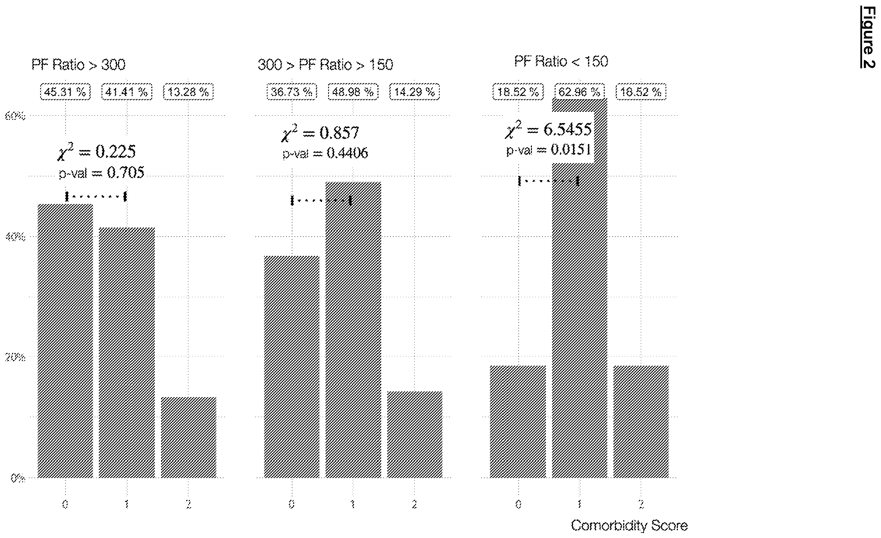

Secondly, as shown by Fig. 2, while there is no significant difference between the proportions of those scoring 0 and 1 on the Comorbidity score, in those with mild (Worst PFR > 300) and those with moderate (300 < Worst PFR < 150) COVID-19 disease, there is a very significant difference in those with the most severe disease (Worst PFR < 150), with a significantly increased proportion of those with severe disease suffering having either diabetes or hypertension (Comorbidity score = 1).

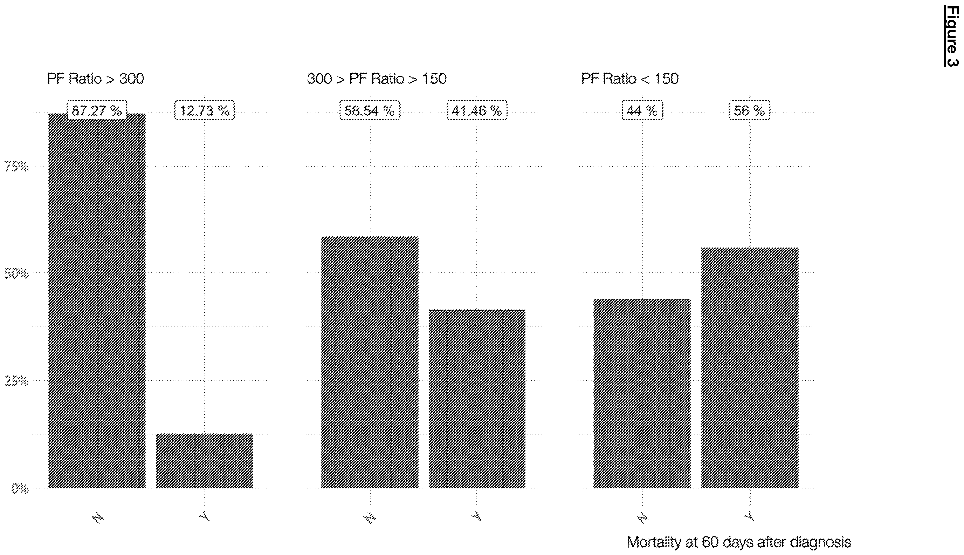

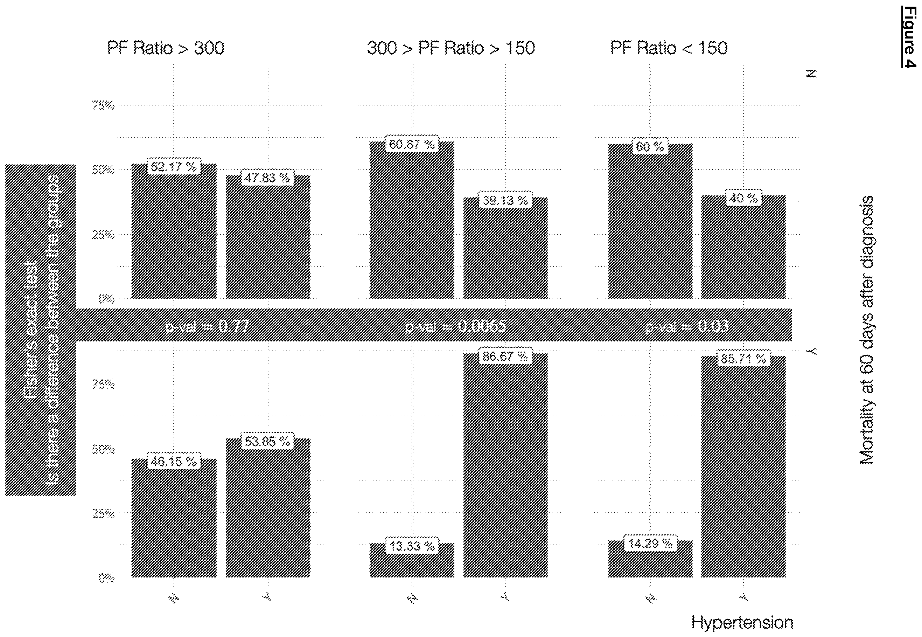

The relationship between disease severity (worst PFR) and mortality (Fig. 3) reveals a predictable trend: an increase in 60-day mortality ("Y") as we travel from those with mild disease (worst PFR > 300) to those with severe disease (Worst PFR < 150), by a difference of 42.27%. When we look at the relationship in 60-day mortality to underlying comorbidities (Fig. 4) we find that the principle driver of increased mortality in those with severe disease (worst PFR < 150) is among those with hypertension. Figure 4 reveals both a large and significant difference between those who died and those who survived at day-60 of disease, in proportion of those with hypertension as a comorbidity, in those with moderate (worst PFR 300 - 150; 47.54%; p = 0.0065) and severe COVID-19 disease (worst PFR < 150; 45.71%; p = 0.03).

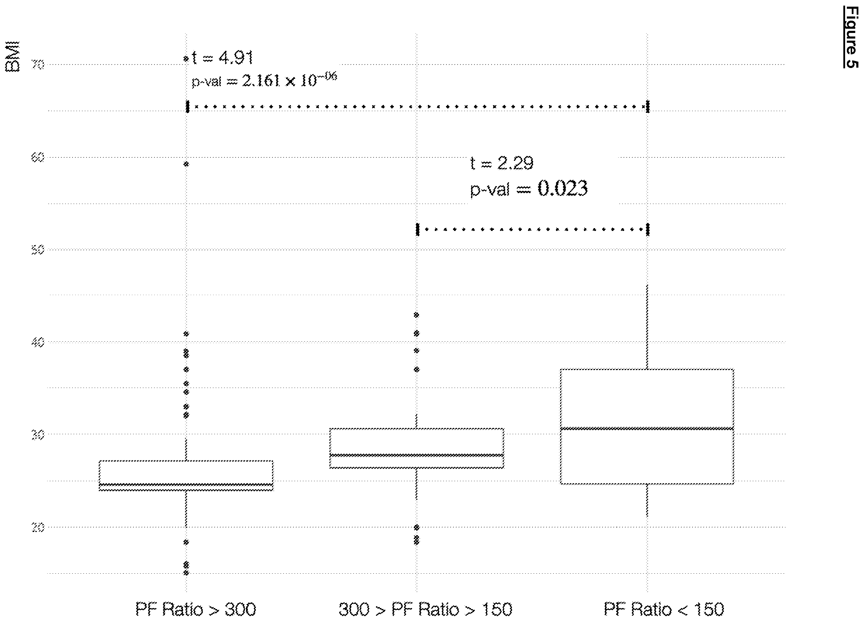

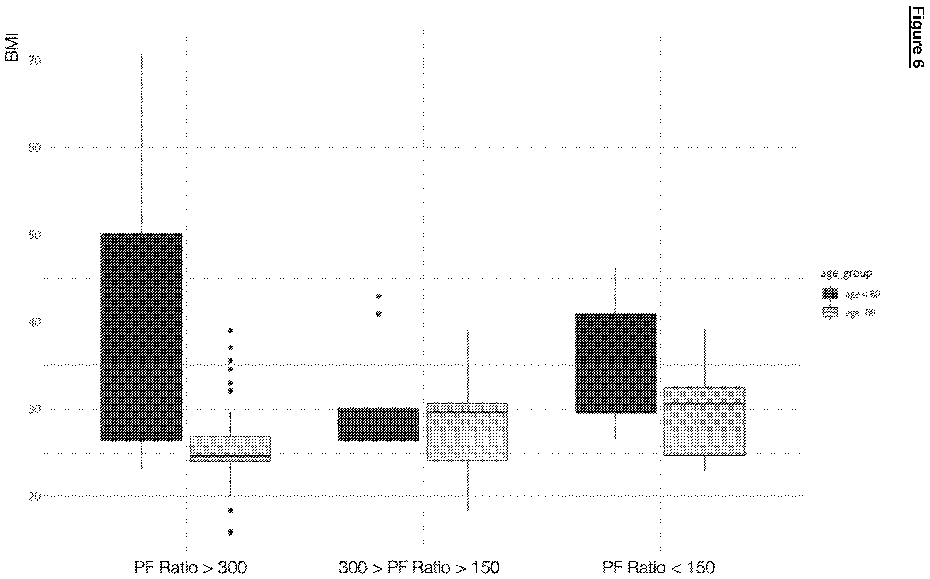

Finally, BMI showed an important and significant difference between those in the severest disease category and those in the mild (p=2.161×10⁻⁰⁶) and moderate (p=0.023) categories of disease severity (Fig. 5). The mean BMI of those with mild disease was below 25, while those in the severest category was above 30. Figure 6 reveals the breakdown of BMI by two age categories, with 60 years of age being the threshold. It reveals a large spread of high BMI patients with mild disease (though still hospitalised) who are at a younger age, skewing the average BMI upwards in that category.

### Free IL18 and disease severity

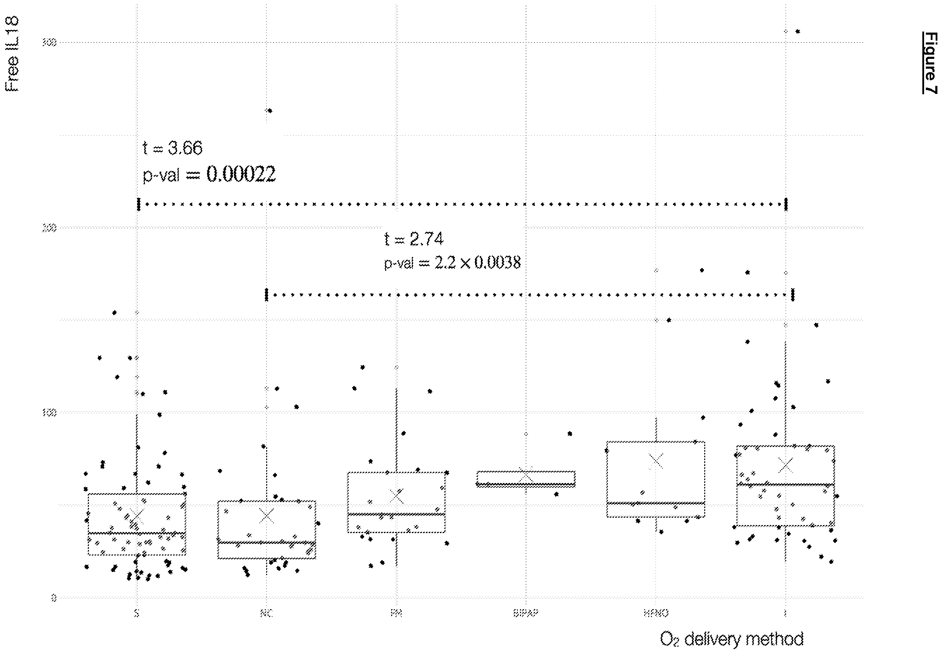

Free IL18 assessed against oxygen delivery method, as a marker of severity, revealed a striking and highly statistically significant difference across the major groups (Fig. 7). Patients hospitalised for COVID-19 but breathing without any oxygen support ("S" - self) or those requiring minimal oxygen support less than 4L/min via nasal cannula ("NC") showed mean Free IL18 levels of 44.01 pg/ml and 44.15 pg/ml respectively, as compared to those intubated ("I") who showed a mean Free IL18 level of 71.31 pg/ml (p=0.00022 S vs I; p=0.008). Similarly, those on emergency oxygen support, as indicated by use of high flow nasal oxygen (HFNO) showed Free IL18 levels of 73.54 pg/ml (p=0.0018 S vs HFNO; p=0.023 NC vs HFNO; not shown on graph).

### Discussion

The data presented here shows a highly statistically significant increase in Free IL18 levels for those requiring high levels of oxygen support ("HFNO" or "I") as compared to those requiring minimal support ("S" or "NC"). This provides good evidence supporting Free IL18 blockade as a treatment modality in viral respiratory pneumonia.

### Comorbidity and Mortality

Our data shows, in consonance with existing studies¹, that comorbidities relating to the metabolic syndrome, hypertension and diabetes, are associated with worse outcomes in COVID-19 disease. In particular, Figure 2 reveals that the percentage of patients without diabetes or hypertension (score 0) falls significantly between those groups with mild disease and those groups with severe disease, as determined by worst PFR, from 45.31% to 18.52%. This is mirrored by a stepwise increase in incidence of diabetes or hypertension (score 1) from the mild group to the severe group, from 41.41% in the mild group to 62.96% in the severe group.

The drive in increased mortality seen with more severe disease also shows a striking association with hypertensive disease, as revealed by Figure 4. Using Fisher's Exact Test, we can see that the proportion of hypertensive patients among those who died by day 60 of their disease course and those who survived, is significantly different, both in the moderate and severe disease groups. The magnitude of the difference is also striking (moderate: 86.67% vs 39.13%; severe: 85.41% vs 40%).

BMI showed an interesting picture, with BMI increasing steadily between mild to moderate to severe groups in a significant manner, between mild to severe and moderate to severe groups (Fig. 5). A closer examination of the BMI by age range (using 60 years as the threshold) showed that BMIs in the mild-severity group were being skewed upwards by a large distribution of younger individuals with high BMI. This indicates the tension that exists between various risk factors such as age and metabolic syndrome; these high BMI patients were at risk of hospitalisation on that account, but were young enough to be protected from severe disease. A closer analysis might reveal, in those under the age of 60 but suffering from severe disease, a difference in their comorbidity profile, to those also under 60 years of age, in the mild-severity group.

### Free IL18 Analysis.

Our data shows a clear relationship between severity of COVID-19 disease, as represented by oxygen delivery methods, and Free IL18 levels. This pattern is consistent across multiple different oxygen delivery method groups, with significant differences between NC or S, and I or HFNO.

It may be asked why severity of disease for the Free IL18 data was not assessed with concurrent PFR rather than method of oxygen delivery, as was done with the mortality and comorbidity data (Figures 2 - 6).

A major reason is that the demographic and comorbidity data involved assessing comorbidity and mortality for all 272 patients against "worst PF ratio" across the entire range of 947 blood-taking events, thus providing a comprehensive assessment of disease severity.

The Free IL18 data, however, was taken from a random selection of blood samples; 211 out of a total selection of 947, from 116 patients out of a total of 247, and so "worst" PFR would not reflect disease severity. Indeed, if the "worst PFR" recorded occurred *before* the Free IL18 blood sampling occurred, the PFR would be actively misleading.

Another option could be PFR concurrent with the blood sampling event. Yet, PFR concurrent with Free IL18 would not provide an accurate assessment of disease severity either, on account of the fact that the blood samples were taken as a random selection from a larger pool, so as to avoid selection bias. Use of concurrent PFR in an incomplete longitudinal data set would thus be misleading; a high Free IL18 may be noted in a patient with a high PFR, with the deleterious effects of the high Free IL18 resulting in *low* PFR only in the *subsequent* days. Without that full longitudinal data set, the effect of the high Free IL18 in driving a later low PFR would go unnoticed. Similarly, a patient may have a high PFR in the recovery phase from severe COVID-19 disease, while still intubated, due to critical care weakness, secondary to an extended period on the ventilator. A high PFR in this case also would obfuscate rather than reveal the severity of the illness the patient had experienced. For these reasons, it was felt that oxygen delivery method would provide a more holistic view of disease severity, given that it represents a holistic trajectory of the patient's clinical course, rather than a snapshot value.

Another important effect of the random sampling is that each different Free IL18 result represents a patient at a different time point in their disease. This explains perhaps, why Free IL18 extends so widely in intubated patients, from a high of 300+ pg/ml down to 40 pg/ml. Different patients at different points in their disease course may have different Free IL18 levels, with those in the early, worsening phase, showing higher levels of Free IL18, and those in the recovery phase from severe disease, showing lower levels. This perspective of the effect of the time course helps also explain why those in the "S" and "NC" groupings also showed a range, though with a lower mean Free IL18 level. Without doing further analysis, we cannot tease out whether such individual with higher Free IL18 level in the "S" group, would go on in the subsequent days from that blood sampling event, to develop significant acute respiratory distress syndrome (ARDS), shifting them into the "HFNO" or "I" groups.

Notwithstanding the attenuating effect of the different time courses on Free IL18 levels, the difference between those on the greatest degree of oxygen support and those on least, is still marked and highly statistically significant. Once contextualised into the patient's individual disease time-course, through completion of all 947 longitudinally-taken blood samples, we hypothesise that the case for blockade of Free IL18 as a novel drug target in modifying the severity of viral respiratory illness, will become even stronger than it already is.

Finally, that patient comorbidity increases with more severe disease, as indicated by Figures 2, 4 and 5, and that Free IL18 also increases in a statistically significant fashion with more severe disease, as indicated by Figure 7, provides sound reasoning for targeting Free IL18 blockade with an IL18 antagonist, specifically in those patients suffering from such comorbidities.

### References

1. Fathi M, Vakili K, Sayehmiri F, Mohamadkhani A, Hajiesmaeili M, Rezaei-Tavirani M, Eilami O. The prognostic value of comorbidity for the severity of COVID-19: A systematic review and meta-analysis study. PloS one. 2021 Feb 16;16(2):e0246190.

## Claims

1. An IL-18 antagonist for use in treating a viral respiratory infection in a subject having metabolic syndrome, coronary artery disease, hypertension, atherosclerosis, type II diabetes or a combination thereof,
wherein the IL-18 antagonist is:
(a) a mammalian or viral IL-18 binding protein that binds free IL-18; or
(b) an antibody that binds specifically to free IL-18.

2. The IL-18 antagonist for use of claim 1, wherein the subject has hypertension.

3. The IL-18 antagonist for use of claim 1, wherein the subject has metabolic syndrome and one or more of coronary artery disease, hypertension, atherosclerosis and type II diabetes.

4. The IL-18 antagonist for use of any one of claims 1 to 3, wherein treating the viral respiratory infection prevents the development of a severe form of a viral respiratory syndrome caused by the viral respiratory infection.

5. The IL-18 antagonist for use of any one of claims 1 to 4, wherein the viral respiratory infection is an infection by a coronavirus, influenza virus, human parainfluenza virus, respiratory syncytial virus, rhinovirus, human metapneumovirus, human bocavirus or adenovirus.

6. The IL-18 antagonist for use of claim 5, wherein the coronavirus is a severe acute respiratory syndrome (SARS) coronavirus or a Middle East respiratory syndrome (MERS) coronavirus.

7. The IL-18 antagonist for use of claim 5 or 6, wherein the coronavirus is SARS coronavirus 2 (SARS-CoV2).

8. The IL-18 antagonist for use of any one of claims 1 to 6, wherein the viral respiratory infection is Coronavirus Disease 2019 (COVID-19), SARS, MERS and/or wherein the viral respiratory infection causes viral pneumonia, viral bronchitis or viral bronchiolitis.

9. The IL-18 antagonist for use of any one of claims 1 to 8, wherein the IL-18 antagonist is human IL-18 binding protein.

10. The IL-18 antagonist for use of any one of claims 1 to 9, wherein the IL-18 binding protein comprises:
(i) an amino acid sequence as set forth in any one of SEQ ID NOs: 1-9;
(ii) an amino acid sequence with at least 70% sequence identity to an amino acid sequence as set forth in any one of SEQ ID NOs: 1-9; or
(iii) an IL-18 binding fragment of (i) or (ii).

11. The IL-18 antagonist for use of any one of claims 1 to 9, wherein the IL-18 binding protein comprises:
(i) an amino acid sequence as set forth in any one of SEQ ID NOs: 1-4;
(ii) an amino acid sequence with at least 70% sequence identity to an amino acid sequence as set forth in any one of SEQ ID NOs: 1-4; or
(iii) an IL-18 binding fragment of (i) or (ii).

12. The IL-18 antagonist for use of any one of claims 1 to 11, wherein the IL-18 antagonist is recombinant human IL-18 binding protein.

13. The IL-18 antagonist for use of any one of claims 1 to 12, wherein:
i. said antibody is a human or humanised antibody and/or a monoclonal antibody;
ii. said antibody is an IgG antibody;
iii. said antibody is an IL-18 binding fragment of an antibody; and/or
iv. said antibody is a Fab', Fab, F(ab')2, single domain antibody, tandem antibody dimer, Fv, scFv, dsFv, ds-scFv, Fd, linear antibody, minibody, diabody, bispecific antibody fragment, bibody, tribody, sc-diabody, kappa(lambda) body, bispecific T-cell engager, dual variable domain immunoglobulin, small immunoprotein, small modular immunopharmaceutical, dual affinity retargeting antibody or a small antibody mimetic comprising one or more CDRs.

14. The IL-18 antagonist for use of any one of claims 1 to 13, wherein the IL-18 antagonist is provided in a pharmaceutical composition comprising a pharmaceutically acceptable diluent or excipient, optionally further comprising one or more additional therapeutically active agents.

15. The IL-18 antagonist for use of any one of claims 1 to 14, wherein:
i. the IL-18 antagonist is parenterally administered; and/or
ii. the IL-18 antagonist is administered intravenously, subcutaneously or via inhalation.

## Patentansprüche

1. IL-18-Antagonist zur Verwendung bei der Behandlung einer viralen Atemwegsinfektion bei einer Person mit metabolischem Syndrom, koronarer Herzkrankheit, Bluthochdruck, Atherosklerose, Typ II Diabetes oder einer Kombination davon,
wobei es sich bei dem IL-18-Antagonisten um:
a) ein Säugetier- oder virales IL-18-Bindungsprotein handelt, das freies IL-18 bindet; oder
b) einen Antikörper handelt, der spezifisch an freies IL-18 bindet.

2. IL-18-Antagonist zur Verwendung nach Anspruch 1, wobei die Person an Bluthochdruck leidet.

3. IL-18-Antagonist zur Verwendung nach Anspruch 1, wobei die Person an einem metabolischen Syndrom und einem oder mehreren von koronarer Herzkrankheit, Bluthochdruck, Atherosklerose und Typ II Diabetes leidet.

4. IL-18-Antagonist zur Verwendung nach einem der Ansprüche 1 bis 3, wobei die Behandlung der viralen Atemwegsinfektion die Entwicklung einer schweren Form eines viralen Atemwegssyndroms verhindert, das durch die virale Atemwegsinfektion verursacht wird.

5. IL-18-Antagonist zur Verwendung nach einem der Ansprüche 1 bis 4, wobei es sich bei der viralen Atemwegsinfektion um eine Infektion durch ein Coronavirus, Influenzavirus, menschliches Parainfluenzavirus, respiratorisches Synzytialvirus, Rhinovirus, menschliches Metapneumovirus, menschliches Bocavirus oder Adenovirus handelt.

6. IL-18-Antagonist zur Verwendung nach Anspruch 5, wobei es sich bei dem Coronavirus um ein Coronavirus des schweren akuten respiratorischen Syndroms (SARS) oder ein Coronavirus des Middle East Respiratory Syndroms (MERS) handelt.

7. IL-18-Antagonist zur Verwendung nach Anspruch 5 oder 6, wobei es sich bei dem Coronavirus um das SARS-Coronavirus 2 (SARS-CoV2) handelt.

8. IL-18-Antagonist zur Verwendung nach einem der Ansprüche 1 bis 6, wobei es sich bei der viralen Atemwegsinfektion um Coronavirus-Krankheit 2019 (COVID-19), SARS, MERS handelt und/oder wobei die virale Atemwegsinfektion virale Lungenentzündung, virale Bronchitis oder virale Bronchiolitis verursacht.

9. IL-18-Antagonist zur Verwendung nach einem der Ansprüche 1 bis 8, wobei es sich bei dem IL-18-Antagonisten um menschliches IL-18-Bindungsprotein handelt.

10. IL-18-Antagonist zur Verwendung nach einem der Ansprüche 1 bis 9, wobei das IL-18-Bindungsprotein Folgendes umfasst:
(i) eine Aminosäuresequenz, wie sie in einer der SEQ ID NRn. 1-9 dargelegt ist;
(ii) eine Aminosäuresequenz mit mindestens 70 % Sequenzidentität mit einer Aminosäuresequenz, wie sie in einer der SEQ ID NRn. 1-9 dargelegt ist; oder
(iii) ein IL-18-bindendes Fragment von (i) oder (ii).

11. IL-18-Antagonist zur Verwendung nach einem der Ansprüche 1 bis 9, wobei das IL-18-Bindungsprotein Folgendes umfasst:
(i) eine Aminosäuresequenz, wie sie in einer der SEQ ID NRn. 1-4 dargelegt ist;
(ii) eine Aminosäuresequenz mit mindestens 70 % Sequenzidentität mit einer Aminosäuresequenz, wie sie in einer der SEQ ID NRn. 1-4 dargelegt ist; oder
(iii) ein IL-18-bindendes Fragment von (i) oder (ii).

12. IL-18-Antagonist zur Verwendung nach einem der Ansprüche 1 bis 11, wobei es sich bei dem IL-18-Antagonisten um rekombinantes menschliches IL-18-Bindungsprotein handelt.

13. IL-18-Antagonist zur Verwendung nach einem der Ansprüche 1 bis 12, wobei:
i. es sich bei dem Antikörper um einen menschlichen oder humanisierten Antikörper und/oder um einen monoklonalen Antikörper handelt;
ii. es sich bei dem Antikörper um einen IgG-Antikörper handelt;
iii. es sich bei dem Antikörper um ein IL-18-bindendes Fragment eines Antikörpers handelt; und/oder
iv. es sich bei dem Antikörper um einen Fab', Fab, F(ab')2, einen Einzeldomänenantikörper, ein Tandem-Antikörperdimer, Fv, scFv, dsFv, ds-scFv, Fd, einen linearen Antikörper, einen Minikörper, einen Diabody, ein bispezifisches Antikörperfragment, einen Bibody, einen Tribody, einen sc-Diabody, einen Kappa(Lambda)-Körper, einen bispezifischen T-Zell-Engager, ein Immunglobulin mit dualer variabler Domäne, ein kleines Immunoprotein, ein kleines modulares Immunopharmakon, einen Antikörper mit dualer Affinität und Retargeting oder ein kleines Antikörpermimetikum handelt, das ein oder mehrere CDRs umfasst.

14. IL-18-Antagonist zur Verwendung nach einem der Ansprüche 1 bis 13, wobei der IL-18-Antagonist in einer pharmazeutischen Zusammensetzung bereitgestellt wird, die ein pharmazeutisch verträgliches Verdünnungsmittel oder einen pharmazeutisch verträglichen Hilfsstoff umfasst und gegebenenfalls weiter ein oder mehrere zusätzliche therapeutische Wirkstoffe umfasst.

15. IL-18-Antagonist zur Verwendung nach einem der Ansprüche 1 bis 14, wobei:
i. der IL-18-Antagonist parenteral verabreicht wird; und/oder
ii. der IL-18-Antagonist intravenös, subkutan oder durch Inhalation verabreicht wird.

## Revendications

1. Antagoniste de l'IL-18 pour une utilisation dans le traitement d'une infection respiratoire virale chez un sujet présentant un syndrome métabolique, une maladie coronarienne, une hypertension, une athérosclérose, un diabète de type II ou une combinaison de ceux-ci,
dans lequel l'antagoniste de l'IL-18 est :
a) une protéine de liaison à l'IL-18 de mammifère ou de virus qui se lie à l'IL-18 libre ; ou
b) un anticorps qui se lie spécifiquement à l'IL-18 libre.

2. Antagoniste de l'IL-18 pour une utilisation selon la revendication 1, dans lequel le sujet présente une hypertension.

3. Antagoniste de l'IL-18 pour une utilisation selon la revendication 1, dans lequel le sujet présente un syndrome métabolique et un ou plusieurs parmi une maladie coronarienne, une hypertension, une athérosclérose et un diabète de type II.

4. Antagoniste de l'IL-18 pour une utilisation selon l'une quelconque des revendications 1 à 3, dans lequel le traitement de l'infection respiratoire virale empêche le développement d'une forme grave d'un syndrome respiratoire viral provoqué par l'infection respiratoire virale.

5. Antagoniste de l'IL-18 pour une utilisation selon l'une quelconque des revendications 1 à 4, dans lequel l'infection respiratoire virale est une infection par un coronavirus, un virus de la grippe, un virus parainfluenza humain, un virus respiratoire syncytial, un rhinovirus, un métapneumovirus humain, un bocavirus humain ou un adénovirus.

6. Antagoniste de l'IL-18 pour une utilisation selon la revendication 5, dans lequel le coronavirus est un coronavirus du syndrome respiratoire aigu sévère (SRAS) ou un coronavirus du syndrome respiratoire du Moyen-Orient (MERS).

7. Antagoniste de l'IL-18 pour une utilisation selon la revendication 5 ou 6, dans lequel le coronavirus est le coronavirus 2 du SRAS (SARS-CoV2).

8. Antagoniste de l'IL-18 pour une utilisation selon l'une quelconque des revendications 1 à 6, dans lequel l'infection respiratoire virale est la maladie à coronavirus 2019 (COVID-19), le SRAS, le MERS et/ou dans lequel l'infection respiratoire virale provoque une pneumonie virale, une bronchite virale ou une bronchiolite virale.

9. Antagoniste de l'IL-18 pour une utilisation selon l'une quelconque des revendications 1 à 8, dans lequel l'antagoniste de l'IL-18 est une protéine de liaison à l'IL-18 humaine.

10. Antagoniste de l'IL-18 pour une utilisation selon l'une quelconque des revendications 1 à 9, dans lequel la protéine de liaison à l'IL-18 comprend :
(i) une séquence d'acides aminés telle que présentée dans l'une quelconque des SEQ ID NO : 1-9 ;
(ii) une séquence d'acides aminés avec au moins 70 % d'identité de séquence avec une séquence d'acides aminés telle que présentée dans l'une quelconque des SEQ ID NO : 1-9 ; ou
(iii) un fragment de liaison à l'IL-18 de (i) ou (ii).

11. Antagoniste de l'IL-18 pour une utilisation selon l'une quelconque des revendications 1 à 9, dans lequel la protéine de liaison à l'IL-18 comprend :
(i) une séquence d'acides aminés telle que présentée dans l'une quelconque des SEQ ID NO : 1-4;
(ii) une séquence d'acides aminés avec au moins 70 % d'identité de séquence avec une séquence d'acides aminés telle que présentée dans l'une quelconque des SEQ ID NO : 1-4; ou
(iii) un fragment de liaison à l'IL-18 de (i) ou (ii).

12. Antagoniste de l'IL-18 pour une utilisation selon l'une quelconque des revendications 1 à 11, dans lequel l'antagoniste de l'IL-18 est une protéine de liaison à l'IL-18 humaine recombinante.

13. Antagoniste de l'IL-18 pour une utilisation selon l'une quelconque des revendications 1 à 12, dans lequel :
i. ledit anticorps est un anticorps humain ou humanisé et/ou un anticorps monoclonal ;
ii. ledit anticorps est un anticorps IgG ;
iii. ledit anticorps est un fragment de liaison à l'IL-18 d'un anticorps ; et/ou
iv. ledit anticorps est un Fab', un Fab, un F(ab')2, un anticorps à domaine unique, un dimère d'anticorps tandem, un Fv, un scFv, un dsFv, un ds-scFv, un Fd, un anticorps linéaire, un minicorps, un diacorps, un fragment d'anticorps bispécifique, un bicorps, un tricorps, un sc-diacorps, un corps kappa(lambda), un activateur bispécifique de lymphocytes T, une immunoglobuline à double domaine variable, une petite immunoprotéine, une petit produit immunopharmaceutique modulaire, un anticorps de reciblage à double affinité ou un petit anticorps mimétique comprenant un ou plusieurs CDR.

14. Antagoniste de l'IL-18 pour une utilisation selon l'une quelconque des revendications 1 à 13, dans lequel l'antagoniste de l'IL-18 est fourni dans une composition pharmaceutique comprenant un diluant ou un excipient pharmaceutiquement acceptable, comprenant facultativement en outre un ou plusieurs agents thérapeutiquement actifs supplémentaires.

15. Antagoniste de l'IL-18 pour une utilisation selon l'une quelconque des revendications 1 à 14, dans lequel :
i. l'antagoniste de l'IL-18 est administré par voie parentérale ; et/ou
ii. l'antagoniste de l'IL-18 est administré par voie intraveineuse, sous-cutanée ou par inhalation.
